# EUROPEAN PATENT APPLICATION

(11) **EP 3 988 521 A1**
(43) Date of publication of application: **27.04.2022**
(21) Application number: 20822864.3
(22) Date of filing: 10.06.2020
(51) Int. Cl.: C07C 13/62, C07C 13/66, A61K 31/56, A61K 31/58, C07J 17/00, A61P 35/00

(54) **CELLULAR SENESCENCE-ACTIVATING COMPOUNDS**

(30) Priority: 10.06.2019 MX 2019006749
(71) Applicant: Universidad Nacional Autónoma De México, México, 04510 (MX); GUAYULERA SAN SALVADOR Y PLANTAS DEL DESIERTO SPR DE RL DE CV, ZACATECAS EL SALVADOR, 98290 (MX)
(72) Inventor: TAVARES SANTAMARIA, Zaira, Ciudad de México, 56210 (MX); MARTINEZ VAZQUEZ, Mariano, Ciudad de México, 14646 (MX); JACOBO HERRERA, Nadia Judith, Ciudad de Mexico, 14643 (MX); ROCHA ZABALETA, Leticia, Ciudad de Mexico, 03740 (MX); ZENTELLA DEHESA, Alejandro, Ciudad de Mexico, 14000 (MX); COUDER GARCIA, Beatriz del Carmen, Ciudad de Mexico, 04370 (MX)
(74) Representative: Igartua, Ismael
(86) International application number: PCT/MX2020/050010
(87) International publication number: WO 2020/251346

(57) **Abstract**

The present invention describes a naturally occurring chemical compound, specifically guayalins A, B, C and D used in medicine having null cytotoxicity and genotoxicity on healthy lymphocyte cells. Said active compounds interfere with the inflammatory process and have antitumoral activity in human cancer cell lines, since they inhibit their growth through a senescence process.

## Description

### TECHNICAL FIELD

The present invention relates to the field of medicinal chemistry, particularly to the therapeutic effects of compounds of natural origin.

### BACKGROUND OF THE INVENTION

Cancer is currently one of the diseases of worldwide greatest concern and a challenge for public health systems, especially for developing countries due to the economic and social aspects that the disease entails.

In Mexico, this disease is an important cause of morbidity and mortality due to nutritional deficiencies and infectious-contagious diseases typical of the region. The ailment remains between the second and third cause of death since 2000 to date. In 2013, cancer caused 12.84% of the deaths registered in Mexico, ranking as the third cause of death only after heart disease (24.3%) and diabetes (14.3%). Among the main types of cancer that have caused 45% of deaths from this disease, between 2000 and 2013, are: lung cancer, stomach cancer, liver cancer, prostate cancer, breast cancer and cervical cancer. The mortality rate from the disease (per 100,000 population) has been observed to be increasing. In 2000 it was estimated at 58.7, while in 2013 the reported mortality rate was 65.1 and it has been calculated that by 2020 this rate will be 79 per 100,000 inhabitants (95% Cl 76.51 to 81.48) and there will be 1,262,861 Mexicans (95% Cl 1,079,419 to 1,446,303) diagnosed with some type of cancer (Mohar-Betancourt et al, 2017).

There are different treatment options for cancer and their use will depend on the type of cancer and the stage of the disease. The most common is to use a combination of surgery with chemotherapy and/or radiotherapy, or also to use immunotherapy treatments, hormonal therapy, or specific target therapy. In Mexico, health institutions include in their clinical practice guidelines different chemotherapy options for the treatment of cancer main types, including:
cisplatin, 5-fluorouracil, oxaliplatin, epirubicin, mitomycin-C, vinorelbine, gemcitabine, capecitabine, paclitaxel, docetaxel, carboplatin, etoposide, tamoxifen, cetuximab, trastuzumab, gefitnib, erlotinib. These drugs have different action mechanisms to inhibit the growth of tumor cells, however, some can have very high costs and others have associated adverse reactions of different degrees of severity. Therefore, in the clinical experience of chemotherapeutic treatment of cancer, two relevant areas of opportunity are frequently presented in the improvement of available treatments: 1) aspects related to the safety and efficacy of the drug; and 2) the cost of available treatments. In general, the cost of chemotherapeutic drugs available in Mexico as well as those also distributed in the world is high. It has been estimated that cancer treatment can exceed US $100,000 per patient in the most advanced stages, which include chemotherapy as well as expenses for surgeries, hospitalizations, among others (Blumen et al, 2016). In addition, significant inter-individual variability has been observed in the response and safety to this type of drug, which may decrease the probability of treatment success in certain cases (Chen et al, 2015; Visa et al, 2018; Zhuang et al, 2017).

Regarding the safety of antineoplastic drugs, those of the cytotoxic type are considered the most toxic drugs that can be prescribed to a human being and among them are the drugs most used in Mexico (for example, cisplatin, 5-fluorouracil, epirubicin, gemcitabine, capecitabine, paclitaxel, etoposide, among others). Many of these have a therapeutic index of 1, which means that the therapeutic dose is practically the same as the toxic dose. Mainly, cytotoxic drugs can affect the bone marrow, gastrointestinal mucosa, and hair follicles because these tissues have a high growth factor, and it is precisely where these drug types exert their action mechanism. Depending on the cytotoxic drug type, can also occur cardiotoxicity, ototoxicity, hepatotoxicity, and nephrotoxicity (Waller and Sampson, 2018). Importantly, the compounds described in the present invention have shown greater safety in toxicological studies performed compared to a reference cytotoxic drug.

The high toxicity of cytotoxic compounds is associated with their action mechanism, since they intervene in the synthesis of cancer cell DNA, but their selectivity is limited since this process is also carried out in non-malignant cells and some of them may have growth rates similar to the malignant ones. The alteration in DNA synthesis generated by cytotoxic drugs can be at different levels, for example: in the biosynthesis of puric or pyrimidic nitrogenous bases, in the formation of ribonucleotides, in DNA biosynthesis, directly on DNA, in the formation of mRNA and/or proteins, or on the synthesized proteins (Waller and Sampson, 2018). On the contrary, the effect of the compounds described in the present invention exert an antitumor effect by promoting a specific cellular senescence process for tumor cells.

Targeted cancer therapies are other pharmacological alternatives for this condition that consist of monoclonal antibodies (bevacizumab, cetuximab, trastuzumab, rituximab) or small inhibitory molecules (erlotinib, gefitinib, imatinib, sorafenib) that differ from the action mechanism used by cytotoxic drugs. In general, targeted therapy drugs have better tolerance than cytotoxic drugs and can be used for different common cancers such as breast, lung, colon, pancreas, lymphoma, leukemia, and multiple myeloma. This type of therapy is particularly aimed at molecules that are expressed in cancer cells, thus representing the beginnings of a personalized therapy. However, it does not seem to be the best option for all patients, since firstly the cancer cells must contain the molecule and/or receptor specific for which the drug is directed, otherwise it could not exert an effect on the tumor. Also, the cost rises a lot in this type of drug; for example, it has been reported that multi-drug treatment for colon cancer that includes bevacizumab or cetuximab can cost up to US $30,790 for an 8-week treatment, while treatment with the same duration, but using fluorouracil with leucovorin has an approximate cost of US $63. Likewise, despite the selectivity of this type of treatment, some adverse reactions to these drugs have already been reported, such as skin rashes, heart failure, thrombosis, hypertension, and proteinuria; in addition, in the case of treatment with small inhibitory molecules, different types of interactions may occur through cytochrome P450 (Gerber, 2008). The compounds described in the present invention are not directed against a specific receptor or protein expressed by the tumor cell, instead, the compounds act on the enzymatic machinery to activate the arrest of the cell cycle of the cells that make up the different types of tumors.

That is why cancer research works have focused on the development of new effective treatments that present a better safety profile and a lower cost than those currently available. In this sense, the use of natural products has become an area of opportunity for the discovery of phytopharmaceuticals with anti-carcinogenic activity since it has been observed that they can interfere with the onset, development, and progression of cancer through the modulation of various cellular mechanisms (proliferation, differentiation, apoptosis, angiogenesis and metastasis). And it has been suggested that the use of natural products can offer a costeffective alternative in the treatment of neoplasms (Rajesh et al, 2015).

Over time, different secondary metabolites of guayule, which means "rubber tree", have been identified and isolated. The *Parthenium argentatum* is a shrub that grows in the arid zones of the north of the country and the south of the United States, which was first mentioned by JM Bigelow in 1852, and its botanical characteristics were described by Asa Gray of Harvard University in 1859 (Hammond and Polhamus, 1965). In this tree the rubber is stored as a colloidal latex suspension confined in individual cells and is practically in all the organs of the plant, stems, roots and leaves (Rollins, 1950). Among the compounds isolated from guayule are partheniol, essential oils (a-pinene, felandral limonene, among others), methoxylated flavonoids, sesquiterpenes called guayulins A, B, C and D from the ethanolic extract, triterpenes of the cycloartan type named argentatines A and C, isoargentine B and argentatine D, among others.

During the chemical process to obtain rubber from guayule, a resin with remarkable performance is obtained since for each kilogram of rubber, one kilogram of resin is obtained, of which argentatines represent 27% (Komoroski et al., 1986).

Argentatines are triterpenes which have been considered in the scientific literature as good candidates for the search for active compounds that interfere with the inflammatory process and have antitumor activity in human cancer cell lines (Akihisa et al, 2000; Dzubak et al, 2006; Flores-Rosete and Martinez-Vazquez, 2008; Oviedo-Chávez et al, 2004; Oviedo-Chávez et al, 2005; Recio et al, 1995a; Recio et al, 1995b; Ukiya et al, 2009).

In particular, the study of argentatines from guayule has shown that these compounds have antimicrobial activity against *Candida albicans, Torulopsis glabrata, Hansenulla sp., Klebsiella pneumoniae* and *Pseudomonas aeruginosa* (Martinez-Vazquez et al, 1994). Likewise, another study evaluated the cytotoxic activity of argentantines A and B in human cancer cell lines (prostate, leukemia, central nervous system, breast, and colon) and it was found that argentatines presented differences in their cytostatic activity in cancer cells, but without showing cytotoxicity or genotoxicity on healthy lymphocytic cells (Parra-Delgado et al, 2005a).

In addition, the growth inhibition capacity of nine compounds derived from argentatines has been evaluated in prostate cancer cell lines, central nervous system, colon, and leukemia, in which it was observed that among these derivatives there are compounds with greater activity in the inhibition of cell growth (Parra-Delgado et al, 2005b); and the anti-inflammatory activity of these and other argentatine derivatives has also been reported in murine models (Romero et al, 2014). In another study, the ability of the argentatine B compound and its derivatives to inhibit cancer cells has been demonstrated (Parra-Delgado et al, 2006) and it has been described that this effect on cancer cells is carried out through a process of cellular senescence (Alcántara-Flores et al, 2015) specifically by acting on the tumor suppressor proteins p53 and p73 of tumor cells (Romero-Benavides et al, 2017). In this last study, it was shown that argentatine B and its derivatives generate an arrest of the cell cycle in the G1 phase and induce the phosphorylation of the aforementioned proteins.

Senescence was proposed by Leonard Hayflick (1961) as the irreversible loss of the proliferative capacity of cells that remain in a metabolically active state necessary for their survival. Cellular senescence can be triggered by different mechanisms that include: cell damage, activation of oncogenes, telomere shortening, with drugs that damage DNA and radiation (Saretzki, 2010).

It is considered that the cellular senescence process *In vitro* can be studied by different methods that determine the loss of DNA replication, the determination of telomere shortening, the increase in the concentration of the β-gaiactosidase enzyme and the expression profile of specific genes. Specific molecular methodologies for the detections of these cellular processes include: incorporation of 5-bromodeoxyuridine or Thymidine-3H, immunohistochemistry for proteins such as PCNA and Ki-67, terminal restriction fragment analysis (TRF) with a radioactively labeled probe that recognizes telomeric repeats, quantification of fluorescence *in situ* hybridization (Q-FISH) for measurement of telomeric fragments, the Flow-FISH technique that combines the properties of Q-FISH with flow cytometry to quantify senescent cells, measurement of enzyme levels β -galactosidase, detection of elements of the signal transduction pathways that maintain senescent phenotypes, genotoxic stress markers, secretion of inflammatory cytokines, among others (Martinez Salazar et al, 2009). All these techniques have advantages and disadvantages and their application depends on the needs of the study; For example, in the state of the art it has been observed that in senescent human fibroblasts there is a significant increase in levels of the β-gaiactosidase enzyme, indicative of telomere shortening, which is why this condition has been considered a reliable biomarker for senescent cells (Dimri et al., 1995; Kurz et al., 2000; Yang and Hua, 2004).

Cellular senescence involves the irreversible arrest of proliferation, resistance to apoptosis and, frequently, the generation of a secretory phenotype of senescent cells characterized by being pro-inflammatory and destroying tissue. Senescent cells accumulate in various tissues during the aging process and are part of the pathogenesis of various chronic diseases, geriatric syndromes, and loss of resilience. That is why it is considered that preventing the accumulation of senescent cells or reducing their load can contribute to the delay, prevention or improvement of multiple conditions associated with senescence (Kirkland and Tchkonia, 2017). However, according to the mechanism of the senescence process, it could be modified to obtain two different therapeutic purposes. One of them would be the already mentioned inhibitory effect of senescent cells to improve the consequences of aging and chronic-degenerative diseases; and the other mechanism would be the induction of a senescence process in tumor cells to exert an antitumor effect.

In documents US 8759304, US 9913851, US 9403866, US 8481721, US 7846904 (D1-D5 respectively) it is explained that certain diseases are associated with a rapid telomeric loss, which results in premature cellular senescence. For example, unlike tumor cells and certain stem cells, somatic cells have little or no telomerase activity and stop dividing when the telomeric ends of at least some chromosomes have been shortened to a critical length, leading to a programmed cellular senescence (cell death). Since the loss of telomeric repeats in somatic cells, which leads to senescence, is increased by low telomerase activity, the induction of telomerase activity, which has the effect of adding arrays of telomeric repeats to telomeres, imparts to deadly somatic cells an increased replicative capacity, and imparts to senescent cells the ability to proliferate and properly exit the cell cycle after repair of damaged tissue.

Telomerase is a ribonucleoprotein that catalyzes the addition of telomeric repeats to the ends of telomeres. Telomeres are long stretches of repeating sequences that cover the ends of chromosomes and are believed to stabilize the chromosome. Telomerase is not expressed in most adult cells, and telomere length decreases with successive rounds of replication. After a certain number of replication rounds, the progressive shortening of telomeres causes cells to enter a stage of telomeric crisis, which in turn leads to cellular senescence.

In this sense, the aforementioned patents describe and protect compounds, their compositions, and methods to increase telomerase activity in cells. Such methods and compositions can be used on cells in cell culture, i.e., *In vitro* or ex *vivo,* or *In vivo,* such as cells growing in tissues of a subject, including human subjects and non-human mammals. Increased telomerase activity promotes cell replication and proliferation capacity, generating an anti-aging effect. On the contrary, the compounds described in the present invention seek to generate a senescence process on tumor cells to prevent their proliferative capacity and thus exert an anti-tumor effect.

In one aspect, the method described in the state of the art comprises identifying a cell or tissue in which an increase in telomerase activity is desired and contacting the cell or tissue with a compound as described in the documents US 7,846,904; US 8,481,721; US 8,759,304; us 9,403,866; US 9,913,851.

The method described in the state of the art includes the identification, determination or diagnosis of a certain condition in a subject in such a way that it is desired to increase telomerase activity in the cells or tissue of the subject, and to administer the compound to the subject. The subject can be a mammalian subject, such as a domestic animal, a dog, or a cat, or also a mouse, a rat, a monkey or a human subject or patient.

Such conditions or diseases for prevention or treatment may include, for example, viral and opportunistic infections, including HIV, various degenerative diseases, such as neurodegenerative diseases, degenerative diseases of the bones or joints and connective tissues, macular degeneration, diabetic retinopathy, cardiovascular diseases, including central and peripheral vascular disease, Crohn's disease and other immune conditions, liver diseases including fibrosis and cirrhosis, lung diseases including pulmonary fibrosis, asthma, emphysema and COPD, hematopoietic disorders (including anemia, thrombocytopenia, neutropenia and other cytopenias), chronic inflammatory disease, gastrointestinal diseases such as Barretts esophagus, as well as any disorder related to the loss of proliferative capacity in stem cell or progenitor cell populations. Such conditions can include bone marrow failure syndrome, aplastic anemia, myelodysplastic anemia, or myelodysplastic syndrome. These conditions also include wounds and other acute or chronic conditions of the skin and its appendages, such as a burn, an abrasion, an incision, a graft, an injury caused by an infectious agent, a chronic venous ulcer, a diabetic ulcer., compression or decubitus ulcer, mucous ulcer, keloid formation, loss of pigment or hair and other structural aberrations of the skin and its appendages. Such conditions also include cancer and precancerous conditions in which low telomerase or shortened telomeres are associated with genomic instability, or increased mutation rates, or loss of tumor suppressor functions, and consequently subjects are at increased risk of tumor initiation, tumor progression or tumor recurrence. However, no experiments or procedures are described in documents D1-D5 that demonstrate the safety of the protected compounds on cancer cells and their safety on healthy cells.

The benefits that can be obtained by increasing telomerase activity in a cell or tissue include, for example, the improvement of the replicative capacity and/or the lifespan of said cell or cells within said tissue.

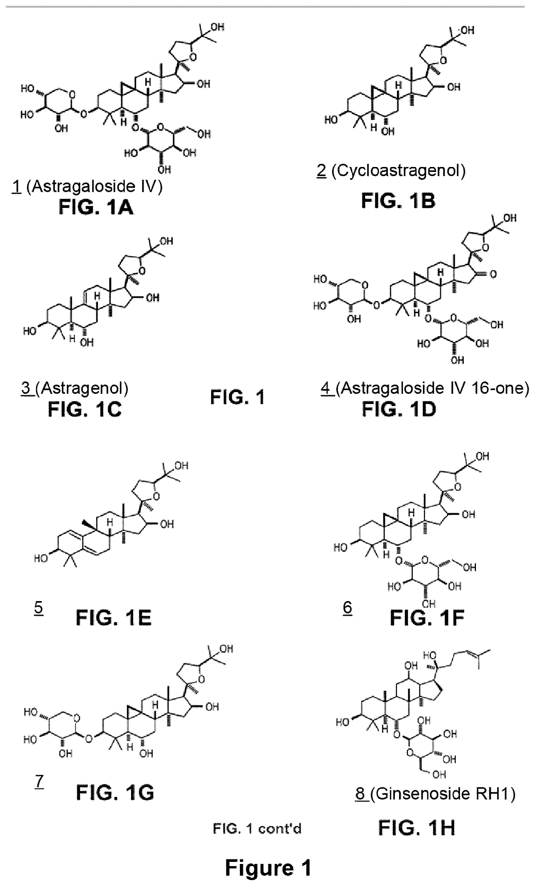

In this sense, for example, in patent No. US 8,759,304 B2 methods and compounds for increasing telomerase activity are protected. These compounds are derivatives of astragalosides with multiple substituents (Figure 1). Astragalosides are alcoholic-type tetracyclic triterpenoids with high polarity (Ren et al, 2013). Conversely, the compound of formula In and its derivatives are triterpenes of the cycloartan type, and although the hydrocarbon skeleton of both structures presents a high similarity, the difference between these compounds lies in an important way in the substituents found in the carbons 3, 6 and 16 which confer different chemical characteristics between astragalosides and cycloartans as in the compound In.

The effect declared for derivatives of astragalosides is to inhibit cellular senescence in healthy somatic cells to promote their proliferation and, therefore, the regeneration of certain cells and tissues that may contribute to the treatment of diseases such as HIV, Alzheimer's disease, heart disease, in transplanted tissues, etc. In a later patent (US 9,913,851 B2), the researchers indicate that the invention can also be applied to cancer and precancerous conditions in which there is a decreased activity of telomerase or short telomeres, which causes genome instability, or an increase in the mutation rate, or loss of the function of tumor suppressor genes and that, consequently, individuals have a higher risk of initiation of tumor formation, progression of an existing tumor, or a recurrent tumor.

However, these studies have not scientifically demonstrated the specific antitumor activity of the protected compounds or their mechanism in animal models for the study of cancer or in tumor cells where the increase in telomerase activity would not lead to a decrease in the proliferation of tumor cells, for which, on the contrary, processes that lead to the induction of a senescence process of tumor cells would be required. The present invention refers to a method that favors the induction of a tumor cell senescence process to inhibit the proliferation of this type of cells and induce their death, through the arrest of the cell cycle. Likewise, as previously mentioned in cancer treatments, their safety depends on the toxicity of the compounds on healthy cells, which is not detailed in the patents found; another difference with respect to the state of the art is that the compounds described in the present invention do not present toxicity on healthy cells, since the compound In and its derivatives act on mechanisms of regulation of the cell cycle that have been indirectly shown to it does not cause an anti-proliferative effect on them.

### BRIEF DESCRIPTION OF THE DRAWINGS

**Figure 1****.** Astragloside compounds used to increase telomerase activity (US Patent 8,759,304 B2).

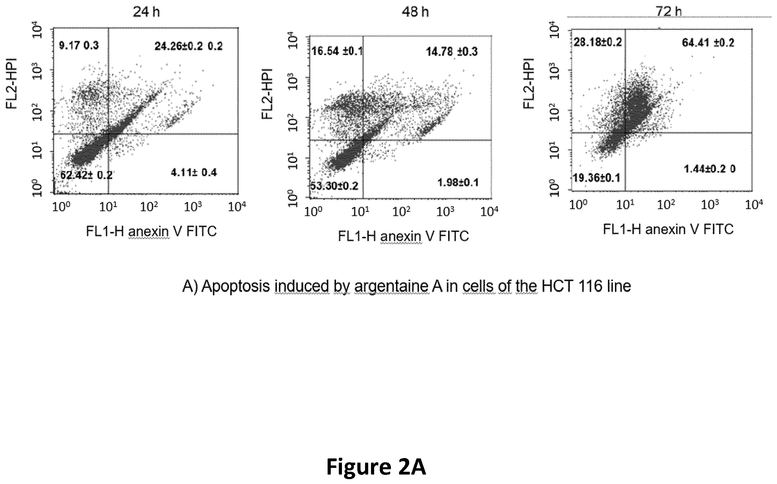

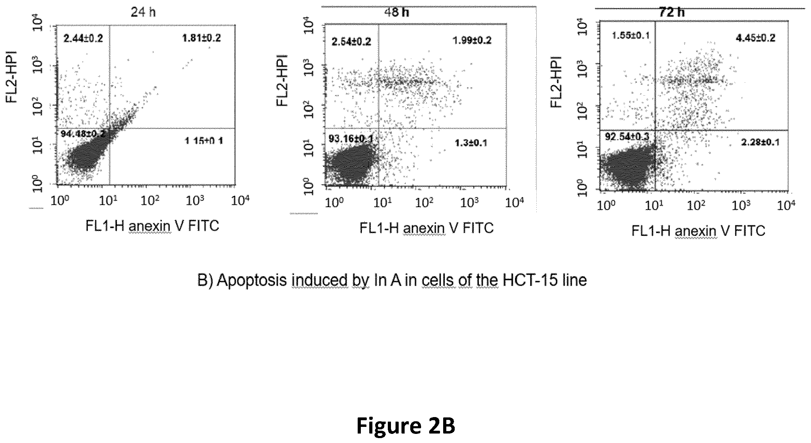

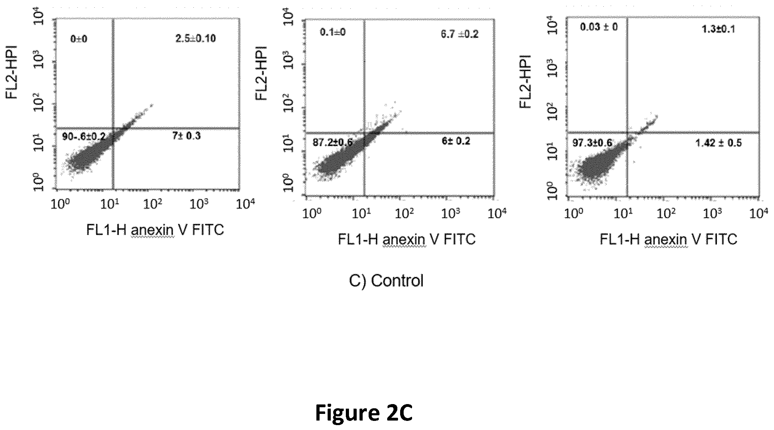

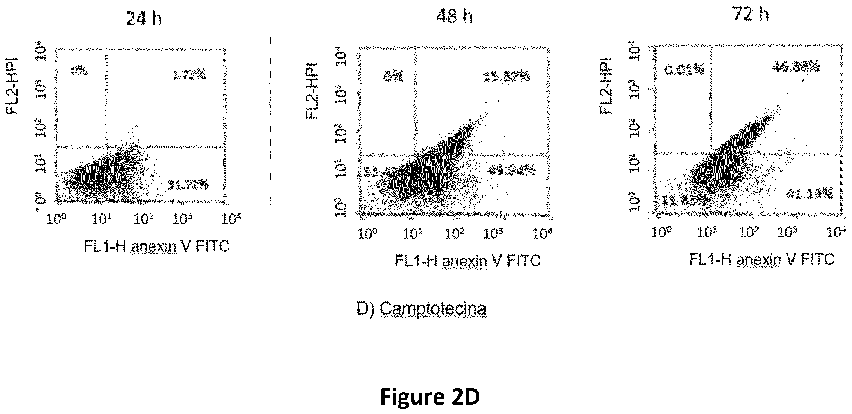

Figure 2.A to 2D Results of the apoptosis induction using a double labeling with annexin V and IP, due to the effect of compound In.

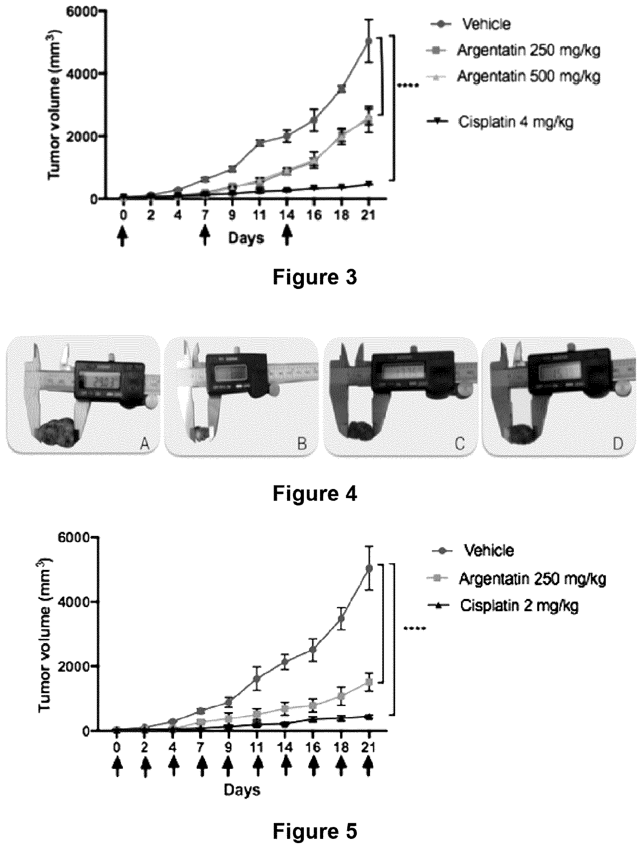

**Figure** 3. Evaluation of the effect of compound In in mice xenotransplanted with HCT-116 cells. The arrows indicate the days of compound administration, in a regimen of 1 administration per week for 3 weeks, each point represents the average and SD of 6 mice. A significant difference between the treated groups was found with In (250 and 500 mg/kg) and the group of mice treated with cisplatin (4 mg/kg) versus the group treated with vehicle (****p <0.0001, Student's t-test).
**Figure 4****.** Images of the size of mouse tumors treated for three weeks with A) vehicle, B) cisplatin 4 mg/kg once a week, C) In 250 mg/kg once a week, D) 500 mg/kg of In once a week. The tumor was induced with the HCT-116 colon cancer cell line.
**Figure 5****.** Evaluation of the effect of compound In in mice xenotransplanted with HCT-116 cells. The arrows indicate the days of administration, with 3 administrations per week for 3 weeks, each point representing the mean ± SD of 6 mice. Significant differences were observed between the In- and cisplatin-treated groups versus the vehicle-treated group (****p <0.0001, Student's t-test).

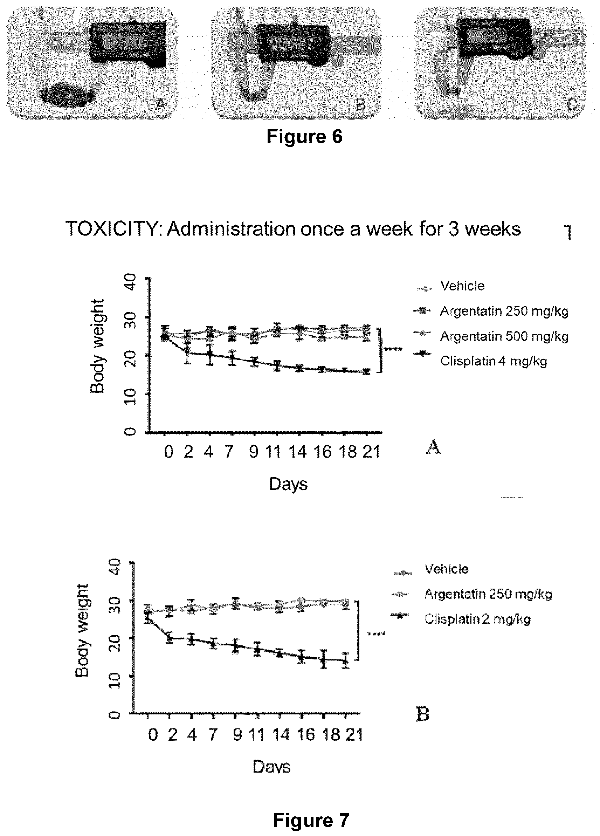

**Figure 6****.** Images of the size of the tumors treated for three weeks with A) vehicle, B) 250 mg/kg of In three times a week, and C) cisplatin 2 mg/kg three times a week.

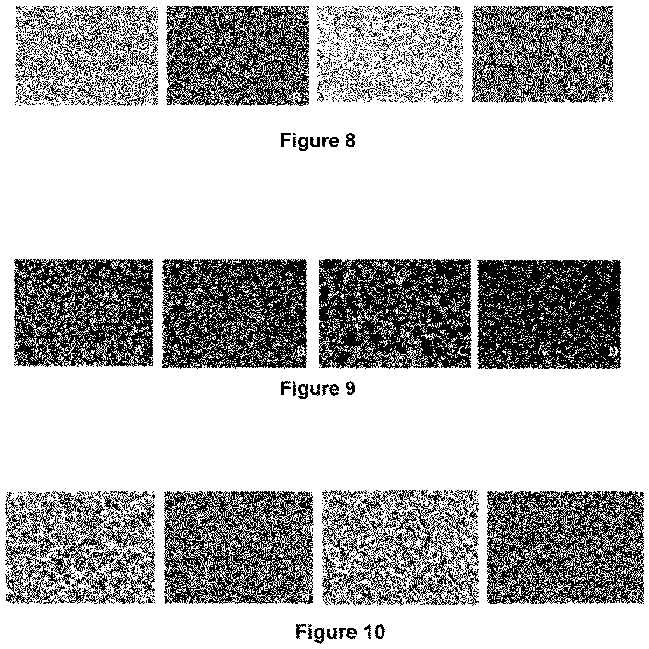

Figure 7. A) Weight variation of nu/nu mice treated with In at doses of 500 mg/kg, 250 mg/kg or cisplatin 4 mg/kg, administered intraperitoneally once a week for 3 weeks. B) Weight variation of nu/nu mice treated with In at doses of 250 mg/kg or cisplatin 2 mg/kg, administered intraperitoneally 3 times a week for 3 weeks. Each point in the graphs represents the mean ± SD of the weight of 3 mice per experimental group. A statistical difference was observed between the weight of the cisplatin-treated mice versus the vehicle group mice (**** p <0.0001, Student's t-test).
**Figure 8****.** Representative photomicrographs of HCT-116 cell xenotransplantation stained with hematoxylin-eosin. A) Vehicle, B) 500 mg of In 1 time per week, C) 250 mg/kg of In 1 time per week, D) 250 mg/kg of In 3 times per week. Images were taken with an Olympus IX71 inverted microscope using Qcapturepro 5 software from the Qlmaging company with a 20x microscopic scale.
**Figure 9****.** Representative photomicrographs of HCT-116 cell xenotransplantation stained with DAPI. A) Vehicle, B) 500 mg/kg of In 1 time per week, C) 250 mg/kg of In 1 time per week, D) 250 mg/kg of In 3 times per week. The images were taken with an Olympus IX71 inverted microscope using Qcapturepro 5 software from the Qlmaging company with a U-mwu2 filter, 330-420 nm excitation band, 400 dichroic mirror with a Fluorite plan 20x NA0.45 objective.
Figure 10. Representative photomicrographs of PCNA immunolabelling. A) Vehicle, B) 500 mg of In 1 time per week, C) 250 mg/kg of In 1 time per week, D) 250 mg/kg of In 3 times per week. Images were taken with an Olympus IX71 inverted microscope using Qcapturepro 5 software from the Qlmaging company with a 20x microscopic scale. Brown color indicates PCNA positive cells.

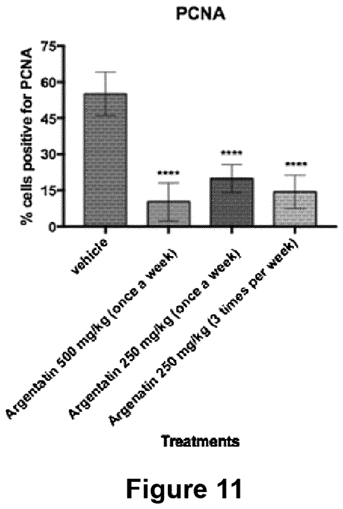

**Figure 11****.** Antiproliferative effect of compound In observed with the cell proliferation marker PCNA in HCT-116 xenotransplanted cells. The results are shown in percentage of PCNA ± SD. The fiji.sc software was used to calculate the average number of antigen-positive cells in 10 randomly selected microscopic fields from 3 xenotransplantation tissues per experimental group, leaving a total of 30 measurements. Significant differences were found between the different treatments with In and the control group (**** p <0.0001, Student's t test).

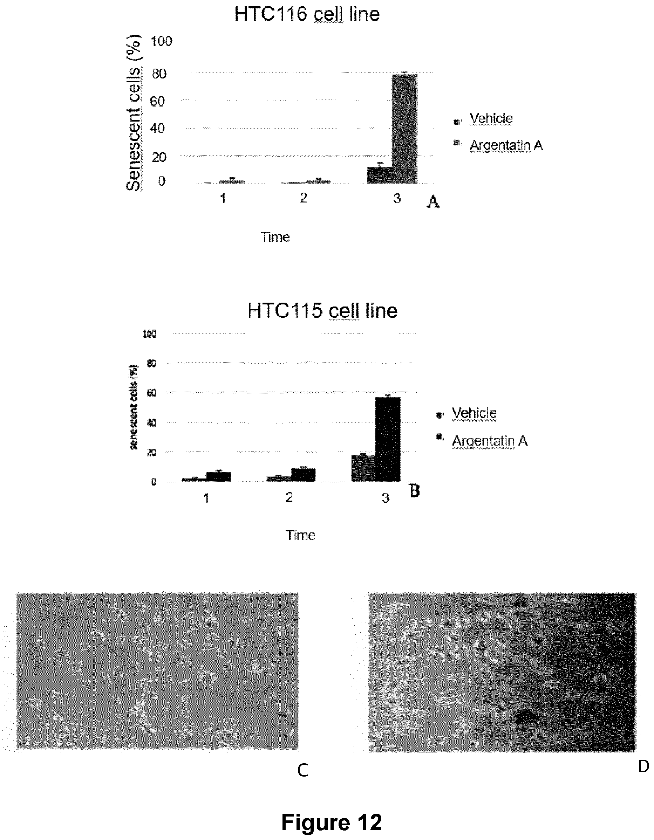

Figure 12. A) β-gaiactosidase activity in HCT-116 cells, B) β-gaiactosidase activity in HCT-15 cells, C) Cell control of the HCT-116 cell line, D) HCT-116 cells treated with In 30 µM for 72 hours.

### DETAILED DISCLOSURE OF THE INVENTION

The present invention relates to a relevant and novel antitumor activity of a compound of formula (I) and (I') with a null toxicity due to the fact that it acts on mechanisms of regulation of the cell cycle that indirectly has been shown not to cause an anti-proliferative effect on healthy cells.

Compound (1) has the formula:

(A) - (B) - (C)

wherein:
A is a group that is selected from one of: 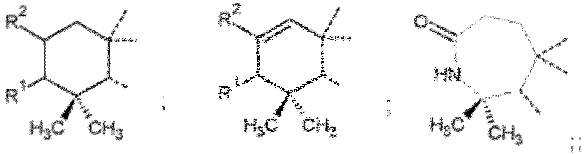
B is 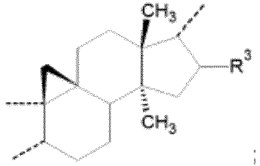 and
C is a group that is selected from one of: 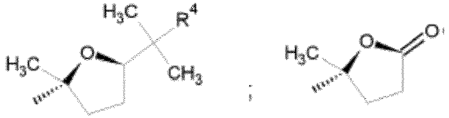
and wherein:
   R¹ represents a group that is selected from: =o 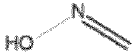 ,-OH;
   R² represents a group that is selected from: -H, -Br, 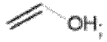
   R³ represents a group that is selected from: -OH, =o, OAc, 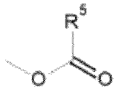
   R⁴ represents a group selected from: -OH, OAC, 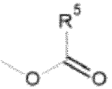
and wherein:
   R¹ and R³ can be at the same time =o;
   R³ and R⁴ can be at the same time -OH or -OAc;
   R¹ and R² are not at the same time 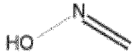 and -Br, respectively;
   R⁵ is a group that is selected from one of: H, CH₃, or an alkyl chain.
and wherein
   when A is 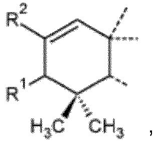 then R¹ is not 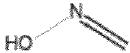 and R² is not -Br, and R³ and R⁴ are at the same time a group selected from -OH or -OAc; or
   when C is -Br; 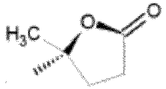 , then R¹ and R³ can be at the same time = O and R² is not or
   when A is 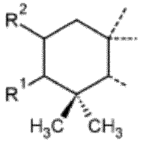 , then R¹ and R² can together form a group 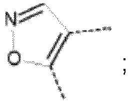
   and enantiomers, diastereoisomers, mixtures of enantiomers, mixtures of diastereoisomers, anomers, hydrates, solvates, polymorphs, of the aforementioned compounds and pharmaceutically acceptable salts thereof.

In one embodiment, a compound of formula (I) is preferred:

(A)-(B)-(C)

wherein:
   A, B, R¹, R², R³, R⁴ and R⁵ have the meanings as defined above,
provided that:
   R³ and R⁴ cannot be at the same time -OH; and
   R¹ and R² are not at the same time =O and -H respectively;
and enantiomers, diastereoisomers, mixtures of enantiomers, mixtures of diastereoisomers, anomers, hydrates, solvates, polymorphs, of the aforementioned compounds and pharmaceutically acceptable salts thereof.

In one embodiment, the compound of formula (I) is a compound wherein:
A is a group 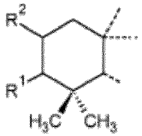
B is a group 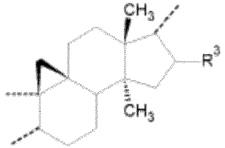
C is a group 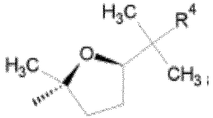
and wherein:
   R¹ represents a group: = O,
   R² represents a group: -H;
   R³ represents a group selected from: -OH;
   R⁴ represents a group selected from: -OH;
and/or the enantiomers, diastereoisomers, mixtures of enantiomers, mixtures of diastereoisomers, anomers, hydrates, solvates, polymorphs, of the aforementioned compound and pharmaceutically acceptable salts thereof.

In one embodiment, a compound of formula (I') is preferred:

(A)-(B)-T

wherein:
   A and B have the meanings as defined above,
   R¹ represents a group that is selected from: =O, 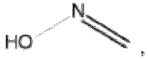 -OH;
   R² represents a group that is selected from: -H,-Sr, 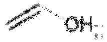
   R³ represents a group that is selected from: -OH, =o, -OAc, 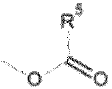
and wherein:
   R¹ and R³ can be at the same time =O;
   R¹ and R² are not at the same time 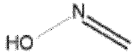 and -Br, respectively;
   R⁵ is a group that is selected from one of: H, CH₃, or an alkyl chain.
and wherein:
   when A is 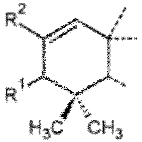 then R¹ is not 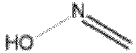 and R² is not -Br; or
   when A is 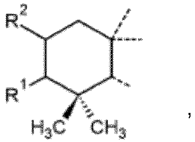 then R¹ and R² can together form a group 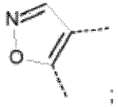 and where T represents a group 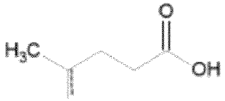
   and enantiomers, diastereoisomers, mixtures of enantiomers, mixtures of diastereoisomers, anomers, hydrates, solvates, polymorphs, of the aforementioned compounds and pharmaceutically acceptable salts thereof.

In one embodiment, compounds of formula (I) and (I') are preferred, which are selected from:

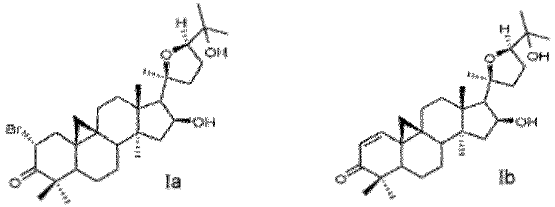

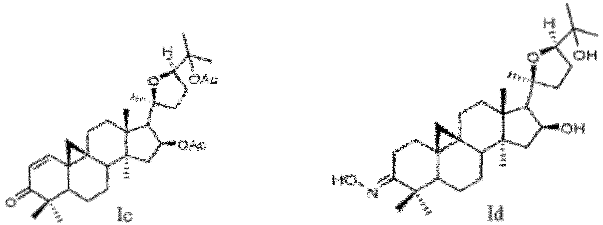

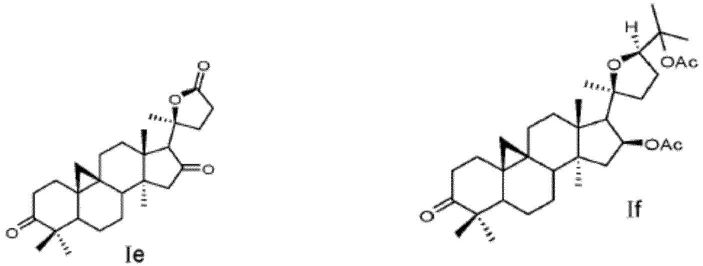

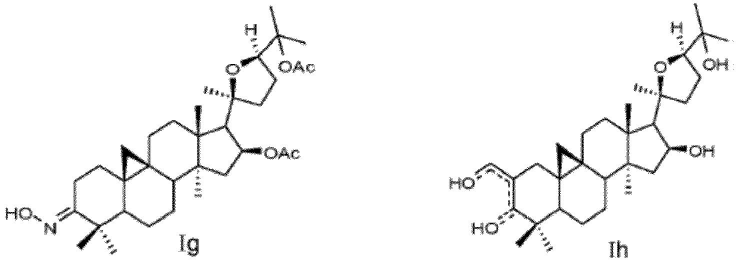

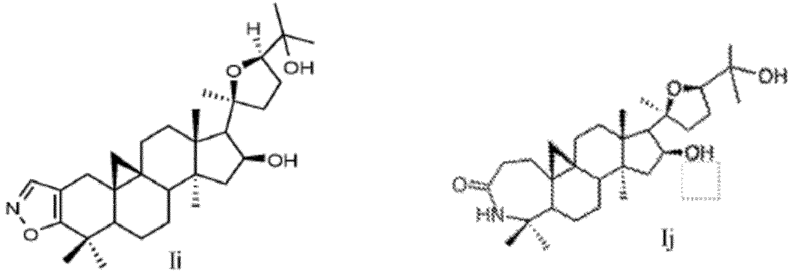

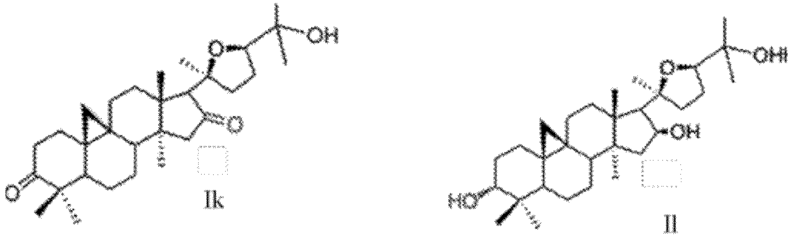

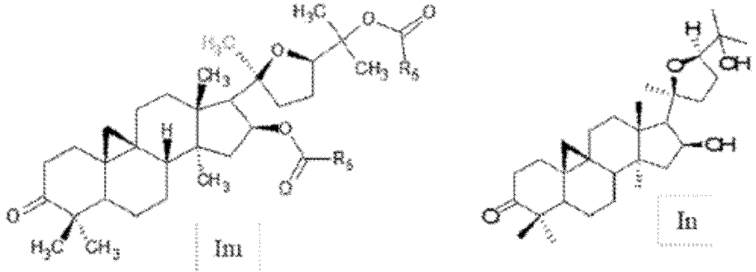

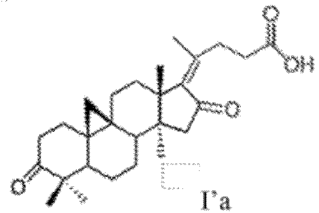

The compound of formula (I) and (I') have an antitumor activity due to the fact that they present null toxicity when acting on mechanisms of regulation of the cell cycle that indirectly has been shown not to cause an anti-proliferative effect on healthy cells in an *in vivo* animal model. Likewise, the compounds of the present invention refer to a method that favors the induction of a senescence process of tumor cells to inhibit the proliferation of this type of cells and induce the death of the same, through the arrest of the cell cycle.

Firstly, the cytotoxic activity of the compounds of formula (I) and (I') was evaluated in a special way of compound In against different colon cancer cell lines (Table 1) where it was observed that the compound exhibits a moderate cytotoxic effect and that the HCT-116 colon cancer cell line was more sensitive to the triterpene administration. Likewise, it was noted that despite the cytotoxic activity of compound In (Argentantine A) on cell lines, it only induced apoptosis in the HCT-116 line (30µM) at 72 hours of treatment (Figure 2). This indicates that the compound In can induce apoptosis in prolonged incubation intervals, however, this is an edge that shows that the compound induces a process of cellular senescence that eventually leads to cell death as will be explained later.

**Table 1. Cytotoxicity of compound In against different colon cancer cell lines under treatment for 24, 48 and 72 hours. Cisplatin was used as a positive control. The presented values correspond to the average of 3 independent experiments performed in triplicate ± standard deviation.**

| Incubation time (h) | Composite, IC₅₀ (µM) ± S.D. | | | | | |
|---|---|---|---|---|---|---|
| | In | | | Cisplatin | | |
| | Cell cancer line | | | Cell cancer line | | |
| | HCT-15 | HCT-116 | SW-620 | HCT-15 | HCT-116 | SW-620 |
| 24 | 95.43 ± 0.6 | 87.83 ± 0.7 | 115.62±0.4 | 12.50±0.5 | 10.81 ±0.7 | 18.03±0.5 |
| 48 | 59.67±1.3 | 53.33±0.2 | 72.46±0.2 | 10.72±0.6 | 8.37±0.5 | 13.87±0.4 |
| 72 | 44.83±0.9 | 43.17±0.4 | 61.33±1.2 | 4.68±0.3 | 3.09±0.3 | 9.09±0.3 |

The National Cancer Institute and some scientific journals indicate that compounds isolated from medicinal plants should be considered cytotoxic agents only when they show ED₅₀ ≤ values 4 µg/mL (mean effective dose). Therefore, the compound In would be considered as an inactive compound according to this reference, however, what is observed in the state of the art is that the compounds of the triterpene type show a low cytotoxicity but an important anti-inflammatory activity that it can exert an important antitumor activity.

In this sense, the antitumor activity of compound In was demonstrated in a xenotransplantation study using mice inoculated with HCT-116 cancer cells. A first study was conducted in which In was administered at doses of 250 to 500 mg/kg of body weight once a week for 21 days and a reduction in tumor size was observed in 49.1% and 48.8%, respectively, compared to the tumor developed by the mouse that did not receive the treatment (Figures 3 and 4).

Subsequently, a study was carried out in which it was shown that with the administration of In at a dose of 250 mg/kg of weight, three times a week, for 21 days, a reduction of 78.1 % of the tumor is achieved compared to the tumor of the untreated mice. This tumor reduction was equivalent to the effect exerted with the administration of 2 mg/kg of cisplatin in the same regimen of administration of compound In (Figures 5 and 6).

In addition to the fact that In is shown to possess an antitumor effect comparable to that of cisplatin, the triterpene-type compound has a very different toxicological profile from that of the drug cisplatin. Firstly, it is shown that the administration of In at doses of 500, 250 or 125 mg/kg, once a week, for 3 weeks does not show toxicity in nu/nu mice, and the calculated mean lethal dose (LD₅₀) was very high greater than 500 mg/kg. Furthermore, the administration of In at doses of 250 and 500 mg/kg does not cause weight loss in mice, contrary to what happened with mice treated with cisplatin at doses of 4 mg/kg once weekly or at doses of 2 mg/kg three times a week for 21 days (Figure 7).

Likewise, In-treated mice (500 mg/kg once a week and 250 mg/kg three times a week for three weeks) show no physical or behavioral changes compared to the control group. However, mice treated with cisplatin (4 mg/kg once a week and 2 mg/kg three times a week for 3 weeks) show evidence of hepatotoxicity (increased alanine aminotransferase and aspartate aminotransferase values) and decreased leukocytes (Table 2).

**Table 2. Blood parameters of the treated mice.**

| _{A} Administration once a week, | | | | | |
|---|---|---|---|---|---|
| _{B} Administration 3 times a week. | | | | | |
| The averages obtained from the 3 mice that made up each group are shown. Significant differences were found in both cisplatin groups (2 mg/kg and 4 mg/kg) when each of them was compared against the control group (*p<0.05, **p<0.001, ****p<0.0001, Student's t test). | | | | | |
| Blood parameters | Reference | In 500 mg/Kg^{A} | In 250 mg/Kg^{B} | Cisplatin 2mg/Kg^{B} | Cisplatin 4mg/Kg^{A} |
| Leukocytes | 3.2-7.0 x10⁹l | 6.5x10⁹l | 7.2x10⁹l | 1.6x1 0⁹1** | 2.1×109l** |
| Lymphocytes | 3.16-7.8x10⁹/l | 6.9x10⁹/l | 7.3x10⁹/l | 0.52 x10⁹/I **** | 0.52x109/l **** |
| Erythrocytes | 7.1-10.2×10¹²/l | 7.29x10¹²/l | 7.4×10¹²/l | 7.14×10¹²/l | 7.36×1012/l |
| Hemoglobin | 149-170 g/l | 158 g/l | 164 g/l | 113 g/L** | 115 g/l** |
| Glucose | 6.6-8.5mmol/l | 7.1mmol/l | 8.0 mmol/l | 7.80 mmol/l | 8.56 mmol/l |
| Urea | 2.6-3.5 mmol/l | 3.2mmol/l | 2.9 mmol/l | 9.06 mmol/l**** | 7.57 mmol/l**** |
| Creatinine | 8.8-26.5µM/l | 20.16 µM/l | 14.2 µM/l | 43.3 µM/l **** | 29.06 µM/l * |
| Alanine aminotransferase | 46-55 UL | 49.6 UL | 53.3 UL | 75.21 UL* | 62.89 UL* |
| Aspartate aminotransferase | 85-101 UL | 98.96 UL | 91.07 UL | 197.32 UL**** | 151.25 UL **** |

Additionally, studies were carried out to evaluate the effect of In administration on cell morphology in tumor tissues stained with hematoxylin-eosin. This shows that the tissues treated with the compound show slightly elongated nuclei and greater space between them, which suggests an expansion of the cytoplasmic area. This finding shows that the cells are suffering some type of damage compared to the tissues of the control group (Figure 8).

To evaluate whether these nuclei are fragmented as a signal of cell death, the tissues were labeled with the fluorescent marker (CAPI (4',6-diamino-2-phenylindole) which binds strongly to regions rich with adenine and thymine in DNA sequences, which is excited with ultraviolet light and detected with a blue filter through fluorescence microscopy (absorption maximum at 358 nm in the ultraviolet range, and its emission maximum is at 461 nm in the blue color spectrum). In this case, it was observed that the treated groups and the control group present similar patterns, that there are no fragmented nuclei that indicate cell death, however, they are noticed more elongated in the tissues from the groups treated with In (Figure 9).

Meanwhile, the inhibition of cell proliferation caused by the compound In was demonstrated by a decrease in cells positive for the expression of proliferating cell nuclear antigen (PCNA) both in the tissues of the mice treated with In at doses of 500 mg/kg once a week as in the tissues of mice treated with the compound at a dose of 250 mg/kg 3 times a week (Figure 10).

The quantification of PCNA confirmed that in the groups treated with compound In, at different doses, there is a decrease in cell proliferation compared to the control group 10-20% vs 54.81% ± 15.63) (Figure 11).

Now, as already mentioned above, the mechanism by which In exerts an antitumor effect without generating cytotoxicity on healthy cells is through the induction of cellular senescence. This process is demonstrated by the evaluation of the activity of the β-gaiactosidase enzyme in cells of colon cancer cell lines. The activity of this enzyme is observed predominantly in cells of the HCT-116 line treated with In for 72 hours at a concentration of 30 µM (Figure 12).

Therefore, with the results obtained, it can be established that compound In has an antitumor effect similar to that exerted by cytotoxic drugs currently on the market but with a marked safety advantage by not producing the same side effects as the available drugs, since this is done through the induction of a process of cellular senescence, where the tumor cell remains in a state of arrest of the cell cycle that consequently leads to cell death, without causing direct damage to healthy cells. The induction of the cellular senescence process in tumor cells can also be associated with derivatives of compound In (la-li) which are described below.

On the other hand, a study of the effect of compounds la-li on different cell lines is reflected in the following table.

**Table 3.**

| **Compound** | **Cl₅₀ ± EE, µM** | | | |
|---|---|---|---|---|
| | **HCT-15 (colon)** | **K562 (leukemia)** | **PC-3 (prostate)** | **U251 (CNS^{&})** |
| **In** | 31.70 ±1.10 | 38.61 ±4.47 | 20.22 ±3.44 | 27.34 ±1.00 |
| **Ia** | 3.23 ±1.10 | 4.34 ±0.75 | 11.06 ±0.66 | 13.89 ±0.44 |
| **Ib** | 16.66 ±1.50 | 16.84 ±3.46 | 15.26±1.70 | 18.88 ±0.36 |
| **Ic** | >100 | >100 | 13.93 ±0.324 | 58.44 ±4.00 |
| **Id** | 35.80 ±1.98 | 44.43 ±5.57 | 46.91 ±2.75 | 26.79 ±2.39 |
| **Ie** | >100 | >100 | >100 | >100 |
| **If** | 40.68 ±2.49 | 68.39 ±8.30 | 22.69 ±2.50 | 30.51 ±3.45 |
| **Ig** | 28.87 ±3.17 | 15.18±2.35 | 12.56 ±2.42 | 21.37 ±1.67 |
| **Ih** | 9.82 ±0.21 | 14.38 ±0.78 | 5.69 ±0.05 | 5.88 ±1.03 |
| **Ii** | 10.24 ±0.77 | 11.02 ±1.05 | 13.00 ±3.56 | 11.30 ±1.55 |

While in the case of compound Im, the % of growth inhibition in different cell lines such as colon (HCT-15), breast (MCF-7), CNS glial cells (U-251), prostate (PC-3), lung (SKUL) and promyelocytic leukemia (K-562), yields the following results:

**Table 4.**

| | % growth inhibition (50 µM) | | | | | |
|---|---|---|---|---|---|---|
| Compound | PC-3 | U251 | HCT-15 | K562 | SKUL | MCF7 |
| In | 20.2 | 27.3 | 31.70 | 38.6 | 0.0 | 26.6 |
| Im | 14.7 | 54.0 | 37.69 | 36.9 | 19.2 | 2.98 |

### Synthetic approach:

### Synthesis of the compound (Ia)

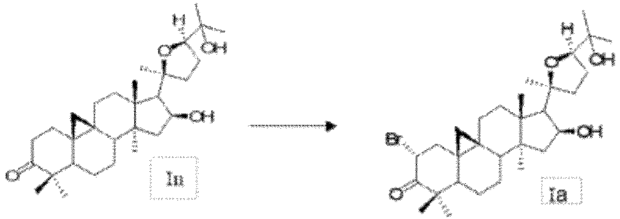

100 mg of In was dissolved in 5 mL of glacial acetic acid and reacted with 0.4 mL of a 1 M solution of bromine in acetic acid. The reaction was carried out under stirring at 3°C. After 1.25 h the reaction mixture was poured into an Erlenmeyer flask containing 50 g of ice. The presence of an abundant precipitate was observed, which was washed with a 5% NaHCO₃ solution and subsequently recrystallized to obtain the product la, (94%). P. f. 116-118°C. IR (film)vₘₐₓ cm⁻¹: 3380.48 (O-H), 2972.39-2872.9 (C-H), 1721.94 (C=O), 1463.42, 1382.14. EMIE m/z (%): 550 (M⁺, 0.77), 498 (15), 496 (15), 351 (5), 349 (5), 143 (100), 125 (30), 107 (31), 81 (20), 71 (32), 43 (30). RMN ¹H (200 MHz, CDCl₃) δ ppm: 0.60 (d, J=4.3, 1 H, H-19), 0.73 (d, J=4.3, 1H, H-19'), 0.89 (s, 3H, CH₃), 1.14 (s, 3H, CH₃), 1.16 (s, 6H, 2CH₃), 1.25 (s, 3H, CH₃), 1.29 (s, 3H, CH₃), 2.70 (sa, 2H, 2 O-H), 3.86 (t, J= 7.7, 1 H, H-24), 4.63 (m, 1 H, H-16), 5.11 (dd, J= 6.5, J= 12.8, 1H, H-2β). RMN¹³C (75 MHz, CDCl₃) δ ppm: 32.9 (C-1), 37.6 (C-2), 201.4 (C-3), 50.9 (C-4), 47.7 (C-5), 21.01 (C-6), 25.6 (C-7), 43.5 (C-8), 20.8 (C-9), 25.9 (C-10), 26.6 (C-11), 31.8 (C-12), 46.2 (C-13), 46.5 (C-14), 48.3 (C-15), 73.2 (C-16), 55.4 (C-17), 21.6 (C-18), 30.2 (C-19), 87.0 (C-20), 21.1 (C-21), 37.3 (C-22), 27.3 (C-23), 84.5 (C-24), 70.9 (C-25), 27.3 (C-26), 26.1 (C-27), 20.4 (C-28), 20.7 (C-29), 21.0 (C-30).

### Compound synthesis (Ib)

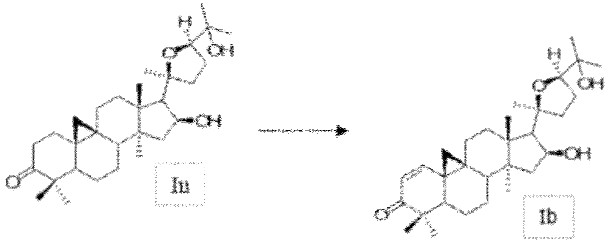

A solution of In (200 mg) and phenylselenium chloride (120 mg) in EtOAc (4.6 ml) was stirred at room temperature for 2 h. Then, 1 mL of water was added to the reaction mixture with stirring. The aqueous phase was separated and 2 mL of THF and 0.2 mL of 30% H2O2 were added. The resulting mixture was stirred at room temperature for 1 hr. After that time, the reaction was processed by conventional methods and an impure solid was obtained. The solid was purified by column chromatography to obtain 140 mg (70%) of the desired product as a crystalline solid of melting point 196-198°C. IR (Film)vₘₐₓ cm⁻¹: 3380.47 (O-H), 2967.73, 2873.35, 1667.21 (C=O), 1463.32, 1379.72. EM-IE m/z (%): 470 (M⁺, 1), 452 (12), 434 (7), 411 (2), 143 (100),125 (25), 107 (10), 59 (10). RMN ¹H (200 MHz, CDCl₃) δ ppm: 0.81 (d, J=4.6, 1H, H-19), 0.90 (s, 3H, CH₃), 0.97 (s, 3H, CH₃), 1.11 (s, 3H, CH₃), 1.15 (s, 3H, CH₃), 1.26 (s, 3H, CH₃), 1.30 (s, 3H, CH₃), 1.42 (s, 3H, CH₃), 3.86 (t, J= 7.6, 1 H, H-24), 4.61 (m, 1 H, H-16), 5.94 (d, J= 10.0, 1 H, H-2), 6.77 (d, J=10.0, 1H, H-1). RMN ¹³C (75.4 MHz, CDCl₃) δ ppm: 153.7 (C-1), 126.7 (C-2), 205.1(C-3),47.0 15 (C-4), 44.9 (C-5), 19.7 (C-6), 27.6 (C-7), 44.5 (C-8), 24.1 (C-9), 29.9 (C-10), 24.0 (C-11), 32.8 (C-12), 46.1 (C-13), 46.3 (C-14), 47.6 (C-15), 73.2 (C-16), 55.5 (C-17), 21.5 (C-18), 30.8 (C-19), 87.2 (C-20), 25.5 (C-21), 37.5 (C-22), 23.8 (C-23), 84.5 (C-24), 70.9 (C-25), 27.3 (C-26), 26.1 (C-27), 19.8 (C-28), 20.1 (C-29), 19.1 (C-30).

### Synthesis of the compound (Ic)

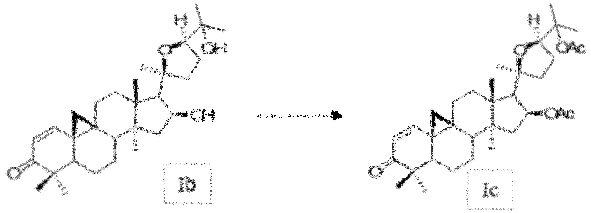

A mixture of 25.5 mg of derivative **Ib,** 21 mg of sodium acetate and 2 mL of acetic anhydride was heated at reflux temperature for one hour. Subsequently, the mixture was poured into an Erlenmeyer flask containing 5 g of ice and stirred for 3 minutes. The contents of the flask were extracted with AcOEt (3x). The organic phase was dried and concentrated under reduced pressure to obtain a semi-solid. Said product was recrystallized (hexane/AcOEt) to obtain 27.4 mg of acetate **Ic** (99%) with a melting point of 158-160°C. IR (Film) cm⁻¹: 2971.64, 2937.91, 2873.33, 1734.33 (C=O), 1668.83 (C=O), 1460.83, 1367.44, 1241.0, 755.46. RMN¹H (300 MHz, CDCl)δ ppm: 0.95 (s, 3H, CH₃), 1.10 (s, 3H, CH₃), 1.22 (s, 3H, CH₃), 1.25 (s, 6H, 2CH₃), 1.47 (s, 3H, CH₃), 1.55 (s, 3H, CH₃), 1.99 (s, 3H, CH₃), 2.03 (s, 3H, CH₃), 2.54 (d, J= 8.49, 1H), 3.74 (t, J= 7.9, 1 H, H-24), 5.40 (m, 1H, H-16), 5.95 (d, J= 10, 1H, H-2), 6.77 (d, J=10, 1H, H-1). RMN ¹³C (75.4 MHz, CDCl₃) δ ppm: 153.5 (C-1), 126.9 (C-2), 205.1 (C-3), 47.0 (C-4), 44.6 (C-5), 19.6 (C-6), 27.8 (C-7), 44.2 (C-8), 24.1 (C-9), 30.1(C-10),23.9 (C-11), 31.8 (C-12), 46.0 (C-13), 46.9 (C-14), 44.6 (C-15), 74.8 (C-16), 56.8 (C-17), 19.4 (C-18), 29.7 (C-19), 85.1(C-20), 22.7 (C-21), 35.1(C-22),25.7 (C-23), 81.7 (C-24), 82.3m(C-25), 28.6 (C-26), 22.9 (C-27), 19.1 (C-28), 19.1 (C-29), 19.1 (C-30), 21.6 and 22.5 (methyl of acetate groups), 170.4 and 170.3 (carbonyl of acetate groups).

### Synthesis of compound (Id)

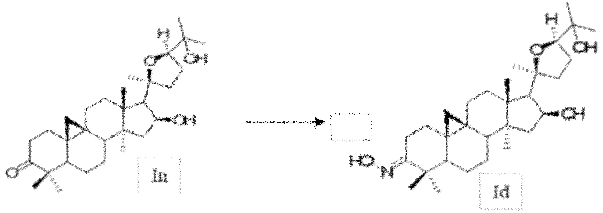

301 mg of compound In in 4.5 mL of pyridine was reacted with 99 mg of NH₂OH·HCl under stirring at reflux temperature for one hour. Subsequently, the reaction mixture was poured into a flask containing 100 g of ice and extracted with AcOEt (3x). The organic phase was repeatedly washed with a 10% HCI solution followed by water and subsequently dried and concentrated under reduced pressure. The residue obtained after evaporation was purified by means of column chromatography, to obtain 277 mg of the oxime **Id** (90%). P. f. 200-205°C. IR (KBr)vₘₐₓ cm⁻¹: 3379.8, 2968.9, 2870.9, 1638.5, 1460, 1380.1, 1103.9. EM-IE m/z (%): 487 (M⁺, 13), 470 (9), 452 (9), 286 (8), 143 (100), 125 (21), 59 (10). RMN ¹H (300 MHz, CDCl₃) δ ppm: 0.54 (d, J=4.2, 1H, H-19), 0.74 (d, J=4.2, 1H, H-19'), 0.88 (s, 3H, CH₃), 1.1 (s, 6H, 2CH₃), 1.3 (s, 3H, CH₃), 1.3 (s, 3H, CH₃), 1.4 (s, 3H, CH₃), 3.38 (dq, 1H),3.9 (t, J= 1.8, J= 7.8, 1 H, H-24), 4.6 (m, 1 H, H-16). RMN ¹³C (75.4 MHz) δ ppm: 32.7 (C-1), 20.0 (C-2), 167.1 (C-3), 43.4 (C-4), 48.8 (C-5), 21.7 (C-6), 26.1 (C-7), 47.6 (C-8), 20.9 (C-9), 27.3 (C-10), 26.1 (C-11), 33.1 (C-12), 46.3 (C-13), 46.6 (C-14), 48.7 (C-15), 73.4 (C-16), 55.6 (C-17), 21.2 (C-18), 30 (C-19), 87.2 (C-20), 25.7 (C-21), 37.3 (C-22), 23.7 (C-23), 84.5 (C-24), 70.8 (C-25), 27.3 (C-26), 26.3 (C-27), 20.3 (C-28), 20.9 (C-29), 20.9 (C-30). A 0.40 x 0.32 x 0.26 mm crystal was used to carry out an X-ray diffraction analysis. Said analysis was performed in a Siemens P4 diffractometer at temperature of 293 K. The compound crystallographic data are shown in Table 5 The experimental conditions and the results of the X-ray diffraction analysis were deposited in the CCDC under the code CCDC 254670.

**Table 5. Id crystallographic information**

| | |
|---|---|
| Empirical formula | |
| Formula weight | 487.7 |
| Crystal system | Orthorhombic |
| Space group | P 2₁ 2₁ 2₁ |
| Unit cell dimensions | |
| A | 27.458 (2) |
| α | 90° |
| B | 7.9900 (10) |
| β | 90° |
| C | 13.0030 (10) |
| γ | 90° |
| Volume Å³] | 2852.9 (2) |
| Z | 4 |
| Density (calculated) [g/cm³] | 1.135 |
| [°] | |
| Index ranges | 0≤ h ≤ 29 |
| | 0≤ k ≤ 8 |
| | 0≤ l ≤ 13 |
| Completeness to θ = | |
| 25.00°[%] | |
| Data/ restraints/ parameters | |
| Goodness-of-fit on *F*² | 1.51 |
| Final R indices [/ > 2σ(/)] | R₁=5.71 |
| | WR₂=7.32 |
| R indices (all data) | R₁=6.16 |
| | WR₂=7.32 |
| Largest diff. Peak and hole | 0.39, -0.26 |
| [e. Å³] | |

### Synthesis of compound (Ie)

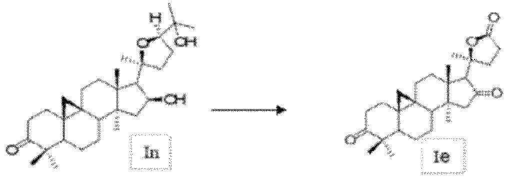

A solution of 100 mg of In in 4 mL of acetic acid was treated at 0-5°C with chromium trioxide (100 mg) in 0.3 mL of water. After 1 hour, the mixture was left at room temperature and subsequently extracted with AcOEt (3x). The organic phase was conventionally processed to obtain the desired product **Ie** (40%). P. f. 138-140°C. IR (Film)vₘₐₓ cm⁻¹: 2972.73, 2876.01, 1768.64 (C=O), 1737.46 (C=O), 1703.84 (C=O), 1462.38, 1385.8. EM-IE m/z (%): 426 (M⁺, 100), 411 (23), 313 (35), 288 (34), 270 (15), 99 (42), 43 (27). RMN¹H (200 MHz, CDCl₃) δ ppm: 0.68m (d, J= 4.5, 1H, H-19), 0.88 (d, J= 4.5, 1H, H-19'), 1.07 (s, 3H, CH₃), 1.12 (s, 3H, CH₃), 1.13 (s, 3H, CH₃), 1.34 (s, 3H, CH₃), 1.49 (s, 3H, CH₃). RMN ¹³C (75.5 MHz) δ ppm: 33.1(C-1),37.3 (C-2), 215.1 (C-3), 50.1 (C-4), 48.5 (C-5), 21.2 (C-6), 26.2 (C-7), 47.0 (C-8), 20.2 (C-9), 26.5 (C-10), 26.1 (C-11), 33.4 (C-12), 45.7 (C-13), 46.2 (C-14), 50.6 (C-15), 215.8 (C-16), 65.1 (C-17), 28.3 (C-18), 30.1 (C-19), 85.5 (C-20), 22.1 (C-21), 42.4 (C-22), 27.8 (C-23), 177.2 (C-24), 19.7 (C-28), 20.7 (C-29), 19.9 (C-30).

### Synthesis of compound (If)

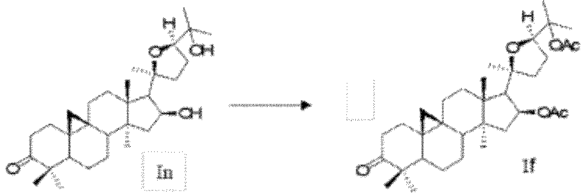

A mixture of 200 mg of In, 60.2 mg of sodium acetate and 5 mL of acetic anhydride was heated at reflux temperature for 18 hours. Subsequently, the mixture was poured into an Erlenmeyer flask containing 50 g of ice and stirred for 15 minutes. Contents of the flask were extracted with AcOEt (3x). The organic phase was dried and concentrated under reduced pressure to obtain a semi-solid. Said product was recrystallized (hexane/AcOEt) to obtain the corresponding acetate **If** (99%) with a melting point of 200-206°C. IR (Film) cm⁻¹ 2971.49, 2943.41, 2872.45, 1734.68 (C=O), 1706.33 (C=O), 1459.92, 1368.46, 1241.62. EM-IE m/z, (%): 556 (M⁺), 496 (24), 436 (27), 395 (28), 185 (52), 143 (42), 125 (100), 43 (57). RMN¹H (300 MHz) CDCl₃ δ ppm: 0.53 (d, J= 4.2, 1 H, H-19), 0.83 (d, J = 4.2, 1H, H-19'), 0.96 (s, 3H, CH₃-21), 1.05 (s, 3H, CH₃-29), 1.09 (s, 3H, CH₃-18), 1.23 (s, 3H, CH₃-26), 1.40 (s, 3H, CH₃-28), 1.47 (s, 3H, CH₃-27), 1.55 (s, 3H, CH₃-21), 2.00 (s, 3H, CH₃), 2.02 (s, 3H, CH₃), 3.74 (t, J= 7.7, 1H, H-24), 5.39 (m, 1H, H-16). RMN ¹³C (300 MHz, CDCl₃): 33.2 (C-1), 37.3 (C-2), 214.9 (C-3), 50.1 (C-4), 47.7 (C-5), 21.2 (C-6), 25.9 (C-7), 48.4 (C-8), 20.7 (C-9), 26.5 (C-10), 26.8 (C-11), 32.1(C-12), 46.6 (C-13), 46.9 (C-14), 45.5 (C-15), 75.04 (C-16), 57.16 (C-17) 20.7 (C-18), 30.3 (C-19), 85.0 (C-20), 22.6 (C-21), 35.2 (C-22), 25.7 (C-23), 81.6 (C-24), 82.3 (C-25), 28.5 (C-26), 22.9 (C-27), 19.9 (C-28), 22.2 (C-29), 20.07 (C-30), 21.5, 22.4, 170.13, 170.29.

### Synthesis of compound (Ig)

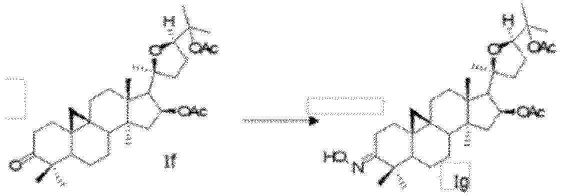

200 mg of the In diacetate in 6.5 mL of pyridine was reacted with 95 mg of NH₂OH·HCl under stirring at reflux temperature for 1.5 hours. Subsequently, the reaction mixture was poured into a flask containing 50 g of ice and extracted with AcOEt (3x). The organic phase was washed 3 times with a 10% HCI solution and then with water. Recrystallization of the organic phase allowed the Ig oxime purification (90%) from p. f. 140-143°C. IR (film)vₘₐₓ cm⁻¹: 3318.57, 2972.66, 2942.16, 2872.86, 1734.27, 1456.29, 1368.98, 1242.09. EM-IE m/z (%): 571 (M⁺), 511 (12), 434 (15), 185 (67), 143 (49), 125 (100), 43 (60). RMN ¹H (300 MHz, CDCl₃) δ ppm: 0.44 (d, J=4.2, 1 H, H-19), 0.72 (d, J=4.2, 1 H, H-19'), 0.94 (s, 3H, CH₃), 1.08 (s, 3H, CH₃), 1.14 (s, 3H, CH₃), 1.22 (s, 3H, CH₃), 1.38 (s, 3H, CH₃), 1.47 (s, 3H, CH₃), 1.54 (s, 3H, CH₃), 2.00 (s, 3H, CH₃), 2.02 (s, 3H, CH₃), 2.52 (d, J= 8.4, 1H, H-17), 3.36 (dc, 1H), 3.73 (t, J= 7.5, 1H, H-24), 5.38 (m, 1H, H-16), 6.62 (sa, 1 H, O-H). RMN ¹³C (75.5 MHz) δ ppm: 32.6 (C-1), 20.0 (C-2), 166.9 (C-3), 42.7 (C-4), 48.7 (C-5), 21.0 (C-6), 25.8 (C-7), 47.5 (C-8), 20.4 (C-9), 26.8 (C-10), 26.5 (C-11), 32.0 (C-12), 46.6 (C-13), 46.9 (C-14), 45.4 (C-15), 75.1 (C-16), 57.0 (C-17), 21.6 (C-18), 30.2 (C-19), 85.1 (C-20), 22.6 (C-21), 35.0 (C-22), 25.6 (C-23), 81.7 (C-24), 82.3 (C-25), 28.6 (C-26), 22.9 (C-27), 19.8 (C-28), 23.6 (C-29), 20.0 (C-30), 21.5 and 22.5 (carbons of the methyls from acetate groups), 170.4 and 170.3 (carbons corresponding to carbonyls in acetate groups).

### Synthesis of compound (Ih)

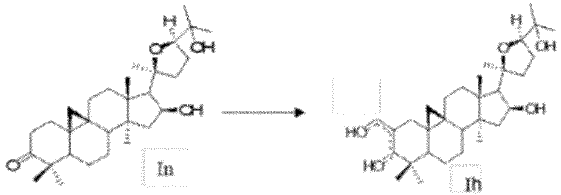

To a solution of 200 mg of In in 6 mL of dry pyridine, contained in inert atmosphere, 1 mL of ethyl formate (freshly distilled) were added, as well as 0.8 mL of a sodium solution in absolute MeOH (0.44 g/6 ml). The reaction was kept stirring at room temperature overnight. The appearance of an ocher color and/or the formation of an insoluble precipitate were considered as evidence of the reaction. After the relevant time, the reaction mixture was placed in a cold solution of 3 mL of acetic acid in 27 mL of water. As a result of the above action, the appearance of a precipitate was observed, which was extracted with methylene chloride. The aqueous phase was discarded. The organic phase was washed with water and extracted with a 2% potassium hydroxide solution. The basic extract was washed with ether, acidified with glacial acetic acid and finally extracted with methylene chloride. The final methylene chloride phase was dried and concentrated under reduced pressure to obtain an impure semi-solid product. Said product was purified by preparative layer chromatography to obtain the formylated derivative **Ih** (89%). IR (KBr)vₘₐₓ cm⁻¹: 3403.7, 2974.0, 2941.9, 2874.8, 1635.1, 1586.9, 1464.6, 1355.9. EM-IE m/z (%): 500 (M⁺, 2), 482 (6), 441 (M⁺-59, 6), 143 (100), 125 (22), 107 (13), 85 (12), 71 (12), 59 (8), 43 (13). RMN ¹H (200 MHz, CDCl₃) δ ppm: 0.49 (d, J= 4.4, 1 H, H-19), 0.70 (d, J= 4.4, 1 H, H-19'), 0.92 (s, 3H, CH₃), 1.1 (s, 3H, CH₃), 1.15 (s, 3H, CH₃), 1.22 (s, 3H, CH₃), 1.26 (s, 3H, CH₃), 1.3 (s, 3H, CH₃), 1.46 (s, 3H, CH₃), 2.60 (d, J= 15, 1H), 3.46 (sa, 2H, 2 O-H), 3.86 (t, J= 7.4, 1H, H-24), 4.63 (m, 1H, H-16), 8.7 (s, 1H, H-31), 14.8 (sa, 1H, O-H). RMN ¹³C (50 MHz) δ ppm: 33.1 (C-1), 106.6 (C-2), 190.5 (C-3), 42.7 (C-4), 48.5 (C-5), 21.4 (C-6), 26.1 (C-7), 44.7 (C-8), 19.3 (C-9), 29.7 (C-10), 25.5 (C-11), 31.8 (C-12), 46.3 (C-13), 46.6 (C-14), 48.6 (C-15), 73.5 (C-16), 55.6 (C-17), 21.2 (C-18), 30.2 (C-19), 87.2 (C-20), 25.2 (C-21), 37.3 (C-22), 23.7 (C-23), 84.5 (C-24), 70.9 (C-25), 27.3 (C-26), 26.1(C-27), 20.6 (C-28), 24.4 (C-29), 21.6 (C-30), 188.9 (C-31).

### Synthesis of compound (Ii)

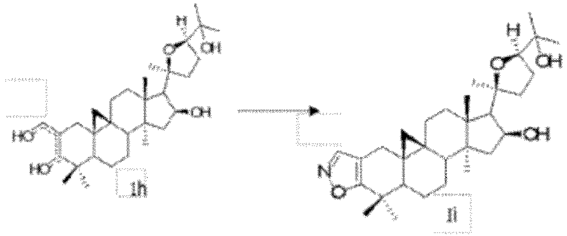

A solution of 60 mg of the formylated derivative Ih in 5 mL of glacial acetic acid, under stirring, was reacted for two hours with 30 mg of hydroxylamine hydrochloride at reflux temperature. The reaction mixture was then poured into an Erlenmeyer flask containing 50 g of ice and extracted with AcOEt. The organic phase was washed with a 5% sodium bicarbonate solution (3x) and with water. The aqueous phases were discarded. The organic phase was dried and concentrated under reduced pressure to obtain an impure semi-solid. Said semisolid was purified by means of column chromatography to obtain the isoxazole **Ii** (80%). P. f. 125-129°C. IR (Film)vₘₐₓ cm⁻¹: 3377.57, 2970.67, 2940.45, 2875.63, 1640, 1564.63, 1463, 1379.57. EM-IE m/z (%): 497 (M⁺, 4), 479 (8), 439 (10), 420 (10), 337 (10), 296 (9), 143 (100), 125 (22), 107 (15), 43 (15). RMN ¹H (300 MHz, CDCl₃) δ ppm: 0.47 (d, J= 4.5, 1 H, H-19), 0.74 (d, J= 4.5, 1 H, H-19'), 0.94(s, 3H, CH₃), 1.15 (s, 15 3H, CH₃), 1.21 (s, 3H, CH₃), 1.26 (s, 3H, CH₃), 1.30 (s, 3H, CH₃), 1.36 (s, 3H, CH₃), 1.46 (s, 3H, CH₃), 2.66 (d, J= 15.6, 1H),3.87 (t, J= 7.2, 1H, H-24), 4.63 (m, 1H, H-16), 7.98 (s, 1H). RMN ¹³C (75.4 MHz) δ ppm: 28.3 (C-1), 109.9 (C-2), 174.8 (C-3), 37.4 (C-4), 48.5 (C-5), 20.8 (C-6), 26.4 (C-7), 46.3 (C-8), 19.7 (C-9), 24.8 (C-10), 25.3 (C-11), 33.2 (C-12), 46.6 (C-13), 46.8 (C-14), 48.8 (C-15), 73.4 (C-16), 55.7 (C-17), 21.2 (C-18), 30.4 (C-19), 87.2 (C-20), 25.7 (C-21), 37.3 (C-22), 23.8 (C-23), 84.5 (C-24), 70.9 (C-25), 27.3 (C-26), 26.1 (C-27), 20.6 (C-28), 25.5 (C-29), 22.2 (C-30), 149.4 (C-31).

### Synthesis of compound (Ij)

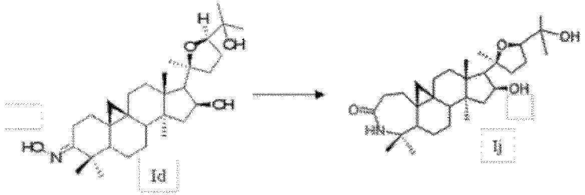

To 100 mg of In oxime dissolved in CHCl₃ 0.5 mL of trifluoroacetic anhydride were added slowly at 0°C. Once the addition was complete, the reaction mixture was kept under constant stirring at 25°C. for 18 min. The reaction mixture was evaporated under reduced pressure. From this reaction, a product identified as In 16-trifluoroaxetoxy-lactam was obtained. A solution of potassium carbonate in methanol was added to said product and it was kept stirring for 15 min. at room temperature, subsequently the solution was filtered, and the solvent was evaporated under reduced pressure. The reaction product was purified by column chromatography with polarity of 2:1 Hex:AcOEt, obtaining 38 mg of **Ij.** IR (CHCl₃): vₘₐₓ cm⁻¹: 3612, 3395, 2963, 2871, 1644 cm⁻¹. EIMS m/z (%): 487 (M⁺, 29.27), 429 (M⁺ -58, 5.4), 428 (11.5), 413 (64.86), 58 (100). HRMS: found *m*/*z* 488.3726, [M+H]+; C₃₀H₅₀NO₄ required 488.3739. ¹H NMR (CDCl₃, 300 MHz): d 0.61 (1 H, d, J = 6 Hz, H-190), 0.68 (1 H, d, J = 6 Hz, H-19), 0.86 (3H, s, H-29130)*, 1.11 (3H, s, H-18), 1.21 (3H, s, H-21), 1.26 (3H, s, H-27), 1.30 (3H, s, H-26), 1.33 (3H, s, H-29/30)*, 3.1 (1 H, m, H-2), 3.83 (1 H, t, J = 7 Hz, H-24), 4.60 (1 H, m, H-16), 7.5 (1 H, s, NH). ¹³C NMR (75 MHz, CDCl₃): d 20.2 (C-18), 20.3 (C-28), 21.0 (C-6), 21.0 (C-9), 22.1 (C-29), 23.9 (C-23), 24.4 (C-30), 25.3 (C-21), 26.5 (C-7), 26.5 (C-10), 26.7 (C-11), 27.0 (C-26), 27.5 (C-27), 29.5 (C-1), 29.9 (C-2), 30.8 (C-19), 33.0 (C-12), 36.2 (C-22), 45.5 (C-14), 45.9 (C-13), 48.2 (C-5), 48.5 (C-8), 50.08 (C-15), 55.5 (C-4), 56.3 (C-17), 69.4 (C-25), 71.5 (C-16), 83.4 (C-24), 85.6 (C-20), 175.6 (C-3).

### Synthesis of compound (Ik)

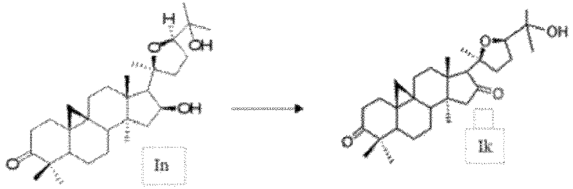

200 mg of In were treated with 170 mg of m-chloroperoxybenzoic acid for 3 hours, to obtain 180 mg of a white solid identified as **Ik.**

### Synthesis of the compound (II)

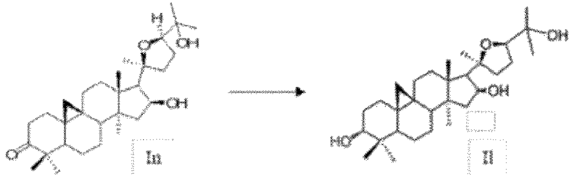

At 100 mg of In, was treated with 80 mg sodium borohydride (NaBH₄), obtaining a white solid identified as **II**, with a melting point of 125-128°C and a molecular weight of 474 g/mol.

### Synthesis of the compound (Im)

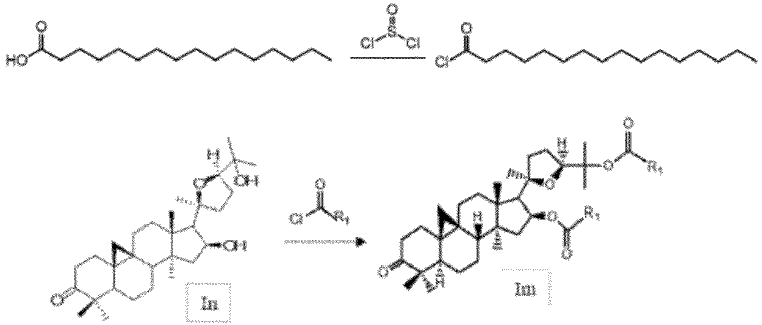

To 0.15 mmol of hexadecanoyl chloride, previously obtained and in inert atmosphere, 0.22 mmol of In was added, dissolved in 4 mL of dry dichloromethane. The reaction was allowed to stir for 30 minutes. After this time, 15 mL of ethyl acetate was added to the reaction mixture, and it was placed in a separatory funnel. The organic phase was washed three times with distilled water and three times with a saturated solution of NaHCO₃, dried with anhydrous Na₂SO₄ and concentrated under reduced pressure. The reaction mixture was subject to silica packed and open column chromatography and. AS elution mixture was used the hexane-ethyl acetate mixture (7:3). From fractions 3-8, was obtained 50 mg of a white amorphous solid with p.f. 246°C with a yield of 70%. IR (CHCl₃ solution) cm⁻¹: 2920.70, 2851.35 (CH), 1732.60 (C=O), 1707.92 (C=O), 1463.77, 1379.20, 1270.56, 1153.83. EM-FAB⁺ m/z (%): 948 (1), 933 (5), 437 (77), 381 (34), 125 (100), 43 (93). EM-IE (70 eV), *m*/*z,* (%): 692 [M⁺- palmitic acid] (6), 436 (24), 381 (22), 256 (22), 125 (100), 43 (54). RMN ¹H (300 MHz, CDCl₃) δ ppm: 5.44 (1 H, ddd J=5.4, J= 2.7 y J=12.3 Hz, H-16), 3.66 (1 H, dd, J=6.9 Hz and J=8.51 Hz, H-24), 1.38 (3H, s), 1.25 (m, for two groups CH₃ and several CH₂ of the palmitate residue), 1.14 (3H, s), 1.09 (3H, s), 1.04 (3H, s), 0.87 (3H, s), 0.81 (1H, d, J= 4.3 Hz, H-19a), 0.59 (1 H, d, J=4.3 Hz, H-19b). RMN ¹³C (75 MHz, CDCl₃) δ ppm: 215.68 (C3), 175.46 and 172.91 (carbonyls of palmitate esters), 81.91 (C24), 81.89 (C25), 74.45 (C16), 56.35 (C17), 49.58 (C4), 47.84 (C8), 47.15 (C5), 46.15 (C13), 46.02 (C14), 44.91 (C15), 35.97 (C2), 36.80 (C22), 33.64 (C1), 32.64 (C12), 29.56 (C19), 29.54 (methylenes from the palmitate residue), 29.01 (C20),26.30 (C11), 25.88 (C7), 27.90 (C26), 27.36 (C27), 24.40 (C23), 21.61 (C21), 21.61 (C18), 20.19 (C29), 19.63 (C30), 14.07 (palmitate methyls).

### Synthesis of the compound (I'a)

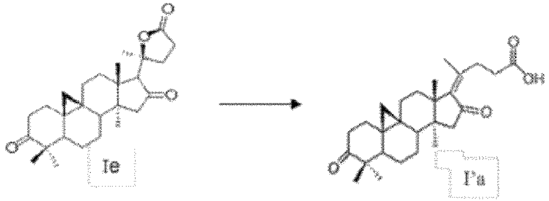

A solution of 100 mg of le in EtOH was reflux maintained with 289 mg of potassium hydroxide for 40 min. After the reaction was neutralized and 70 mg of 3,16-dioxo-25-nor-cycloartan-17-en-24-oic acid [I'a] was obtained. P.f. 182-184°C. IR (KBr): vₘₐₓ cm⁻¹: 3327, 1741, 1703, 1615 cm⁻¹. EIMS *m*/*z* (%): 426 (M⁺, 39.69), 411 (100), 143 (4.5), 125 (3). HRMS: found *m*/*z* 427.2864, [M+H]⁺; C₂₇H₃₉O₄ required 427.2848.¹H NMR (300 MHz, CDCl₃): δ ppm: 0.64 (d, J = 4.2 Hz, 1 H, H-19), 0.86 (d, J = 4.8 Hz, 1 H, H-190), 0.99 (s, 3H, CH₃), 1.06 (s, 3H, CH₃), 1.11 (s, 3H, CH₃), 1.35 (s, 3H, CH₃), 1.92 (s, 3H, CH₃). ¹³C NMR (75 MHz, CDCl₃): δ ppm: 20.2 (C-18), 20.7 (C-28), 20.8 (C-30), 20.9 (C-9), 21.2 (C-29), 22.1 (C-21), 22.6 (C-6), 26.0 (C-7), 26.3 (C-11), 26.3 (C-10), 29.2 (C-23), 30.6 (C-19), 30.8 (C-22), 32.6 (C-12), 33.1 (C-1), 37.2 (C-2), 42.3 (C-13), 45.7 (C-14), 47.9 (C-8), 48.2 (C-5), 50.20 (C-4), 51.0 (C-15), 141.7 (C-17), 149.5 (C-20), 177.8 (C-24), 207.3 (C-16), 216.0 (C-3).

## Claims

1. A guayulin compound A, B, C and D called:
Argentatines A and C,
Isoargentine B and
Argentatine D,
of formula (I) and (I') that present null cytotoxicity or genotoxicity on healthy lymphocytic cells in an *In vivo* animal model, **characterized in that** it presents anti-inflammatory activity, inhibits the growth of cancer cells through a senescence process;
Wherein the compound of formula (I):
(A)-(B)-(C)
wherein:
A is a group that is selected from one of: 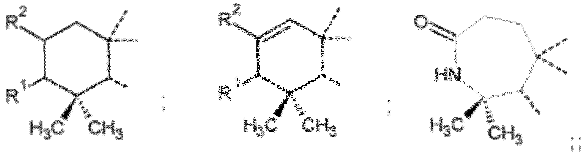
B is 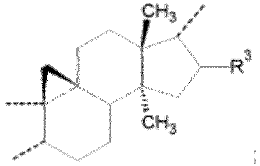 and
C is a group that is selected from one of: 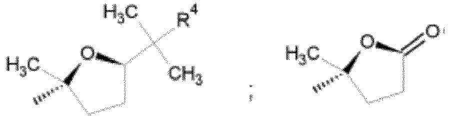 and wherein:
R¹ represents a group that is selected from: 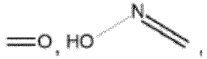 - OH;
R² represents a group that is selected from: -H. -Br, 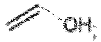
R³ represents a group that is selected from:-OH, =o, -OAc, 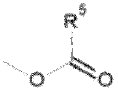
R⁴ represents a group selected from: -OH, -OAc, 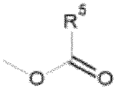
and wherein:
R¹ and R³ can be at the same time =o;
R³ and R⁴ can be at the same time -OH or -OAc;
R¹ and R² are not at the same time 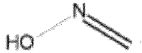 and -Br, respectively;
R⁵ is a group that is selected from one of: H, CH₃, or an alkyl chain.
and wherein
when A is 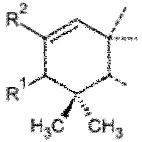 , then R¹ is not 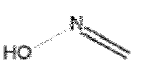 and R² is not -Br, and R³ and R⁴ are at the same time a group selected from -OH or -OAc; or
when C is 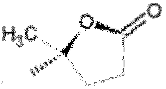 , then R¹ and R³ can be at the same time = O and R² is not -Br; or
when A is 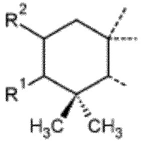 , then R¹ and R² can together form a group 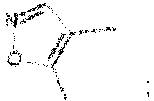 and enantiomers, diastereoisomers, mixtures of enantiomers, mixtures of diastereoisomers, anomers, hydrates, solvates, polymorphs, of the aforementioned compounds and pharmaceutically acceptable salts thereof.
And the compound of formula (I'):
(A)-(B)-T
wherein:
A and B have the meanings as defined above,
R¹ represents a group that is selected from: =O, 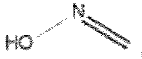 , - OH;
R² represents a group that is selected from: -H, -Br, 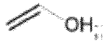
R³ represents a group that is selected from: -OH, =o, -OA_{C} and wherein: 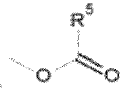
R¹ and R³ can be at the same time =O;
R¹ and R² are not at the same time 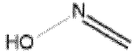 and -Br, respectively;
R⁵ is a group that is selected from one of: H, CH₃, or an alkyl chain, and wherein
when A is 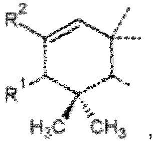 then R¹ is not 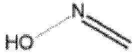 and R² is not -Br;
when A is 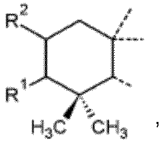 then R¹ and R² can together form a group 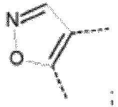 and where T represents a group 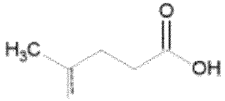
and enantiomers, diastereoisomers, mixtures of enantiomers, mixtures of diastereoisomers, anomers, hydrates, solvates, polymorphs, of the aforementioned compounds and pharmaceutically acceptable salts thereof.

2. A guayulin compound of formula (I):
(A)-(B)-(C)
wherein
A, B, R¹, R², R³, R⁴ and R⁵ is defined in accordance with claim 1, **characterized in that**:
R³ and R⁴ cannot be -OH at the same time; and
R¹ and R² are not at the same time =O and -H respectively;
and enantiomers, diastereoisomers, mixtures of enantiomers, mixtures of diastereoisomers, anomers, hydrates, solvates, polymorphs, of the aforementioned compounds and pharmaceutically acceptable salts thereof.

3. A guayulin compound of formula (I):
(A)-(B)-(C)
according to claim 1, **characterized in that**:
A is a group 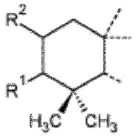
B is a group 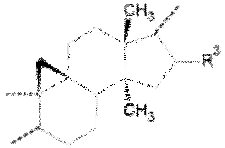
C is a group 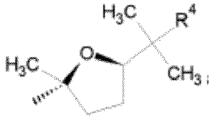
and wherein:
R¹ represents a group: = O,
R² represents a group: -H;
R³ represents a group selected from: -OH;
R⁴ represents a group selected from: -OH;
and/or the enantiomers, diastereoisomers, mixtures of enantiomers, mixtures of diastereoisomers, anomers, hydrates, solvates, polymorphs, of the aforementioned compound and pharmaceutically acceptable salts thereof.

4. A guayulin compound of formula (I') according to claim 1:
(A)-(B)-T
wherein:
A and B have the meanings as defined above, **characterized in that**:
R¹ represents a group that is selected from: =O, 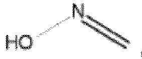 - OH:
R² represents a group that is selected from: -H. -Br, 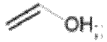
R³ represents a group that is selected from: -OH, =o, -OAc, 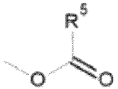
and wherein:
R¹ and R³ can be at the same time =O;
R¹ and R² are not at the same time 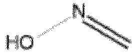 and -Br, respectively;
R⁵ is a group that is selected from one of: H, CH₃, or an alkyl chain, and wherein
when A is 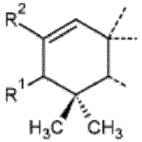 then R¹ is not 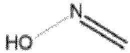 and R² is not -Br; or
when A is 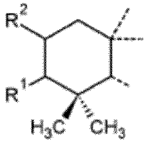 , then R¹ and R² can together form a group 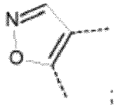 and where T represents a group 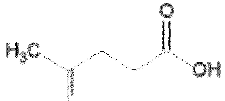
and enantiomers, diastereoisomers, mixtures of enantiomers, mixtures of diastereoisomers, anomers, hydrates, solvates, polymorphs, of the aforementioned compounds and pharmaceutically acceptable salts thereof.

5. A guayulin compound of formula (I) and (I') according to claim 1, **characterized in that** the compounds of formula **(I)** and **(I')** are selected from:

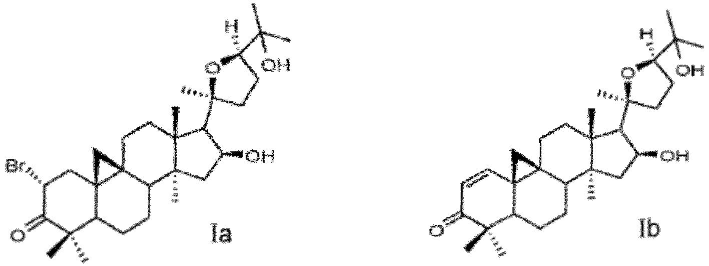

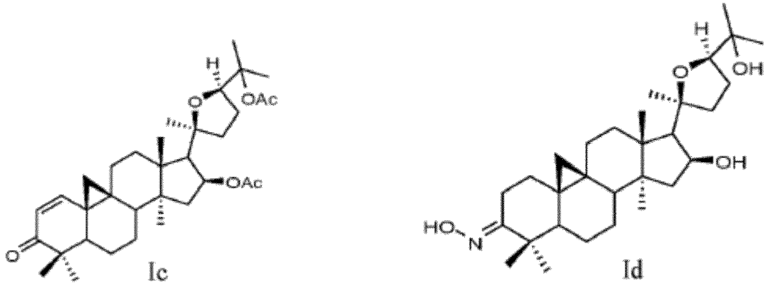

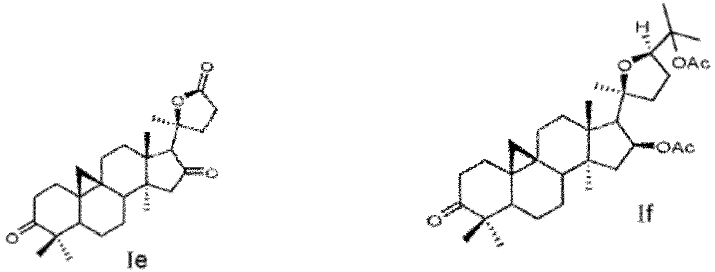

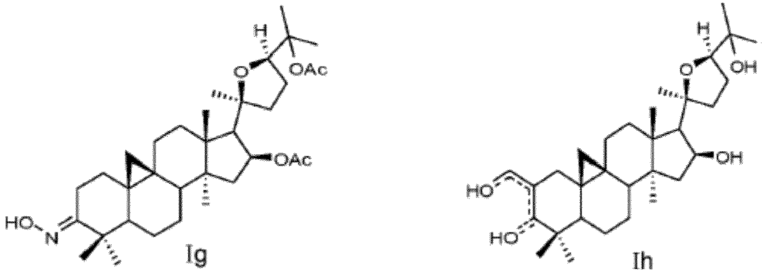

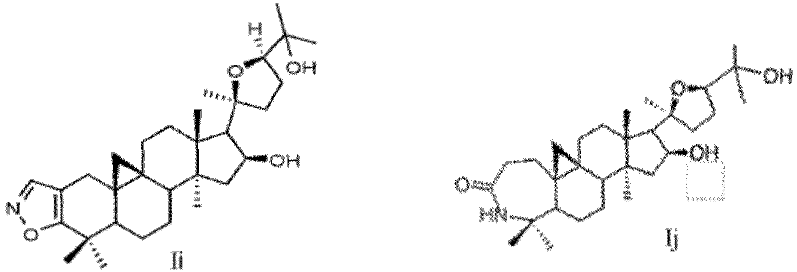

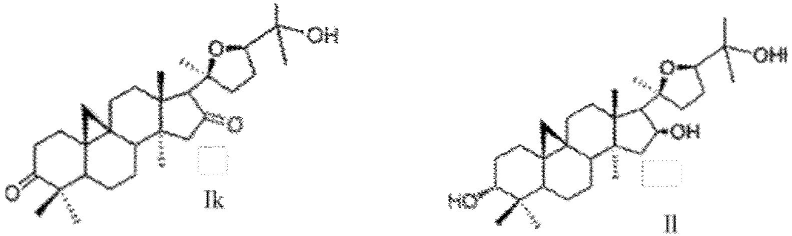

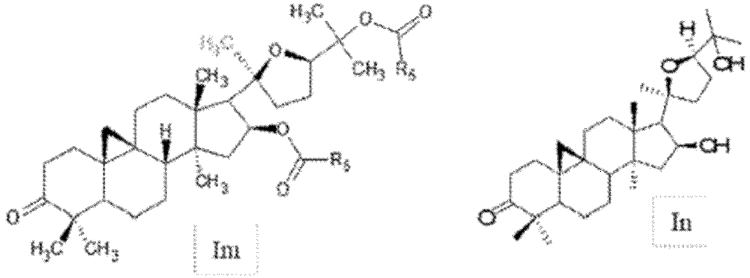

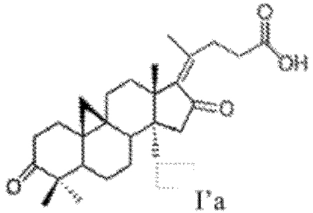

6. A guayulin compound of formula (I) and (I') according to claim 1, **characterized in that** the compound In is the compound

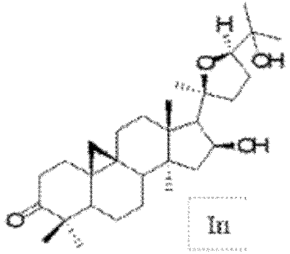

7. A guayulin compound according to claim 5, **characterized in that** the synthesis of the compound **(Ia)** is carried out according to the following steps: 100 mg of In were dissolved in 5 ml of glacial acetic acid reacting with 0.4 ml of a 1 M solution of bromine in acetic acid. The reaction is carried out under stirring at 3°C for 1.25 h, the reaction mixture is poured into an Erlenmeyer flask containing 50 g of ice, washed with a 5% NaHCC>₃ solution and subsequently recrystallized:

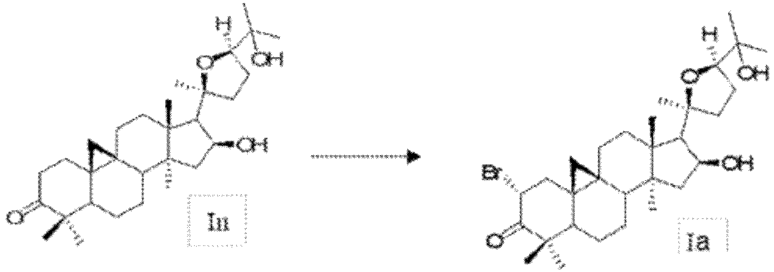

8. A guayulin compound according to claim 5, **characterized in that** the synthesis of the compound **(Ib)** is carried out in accordance with the following steps: a solution of In (200 mg) and phenylselenium chloride (120 mg) in EtOAc (4.6 mL) stirring at room temperature for 2 h; subsequently 1 ml of water is added to the reaction mixture while stirring. The aqueous phase is separated and 2 mL of THF and 0.2 ml of 30% H₂O₂ were added. The resulting mixture is stirred at room temperature for 1 h.

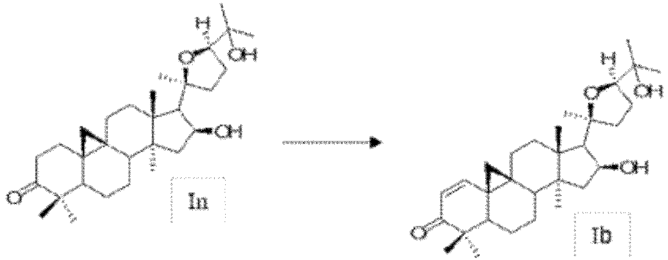

9. A guayulin compound according to claim 8, **characterized in that** the synthesis of the compound **(Ic)** is carried out according to the following steps: a mixture of 25.5 mg of derivative **Ib,** 21 mg of sodium acetate and 2 ml of acetic anhydride is heated at reflux temperature for one hour; subsequently, the mixture is poured into an Erlenmeyer flask containing 5 g of ice and stirred for 3 minutes. The contents of the flask are extracted with AcOEt (3x). The organic phase is dried and concentrated under reduced pressure to obtain a semi-solid; recrystallize and the acetate **Ic** is obtained

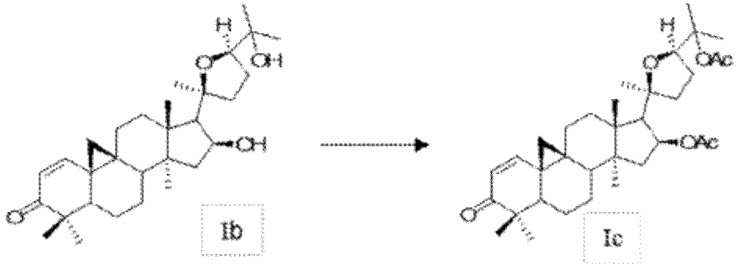

10. A guayulin compound according to claim 5, **characterized in that** the synthesis of the compound (Id) is carried out according to the following steps: 301 mg of compound In in 4.5 ml of pyridine are reacted with 99 mg of NH₂OH·HCl stirring at reflux temperature for one hour; subsequently, the reaction mixture was poured into a flask containing 100 g of ice and extracted with AcOEt (3x). The organic phase is washed repeatedly with a 10% HCI solution followed by water and subsequently dried and concentrated under reduced pressure. The residue obtained after evaporation is purified by means of column chromatography, to obtain the oxime **Id.**

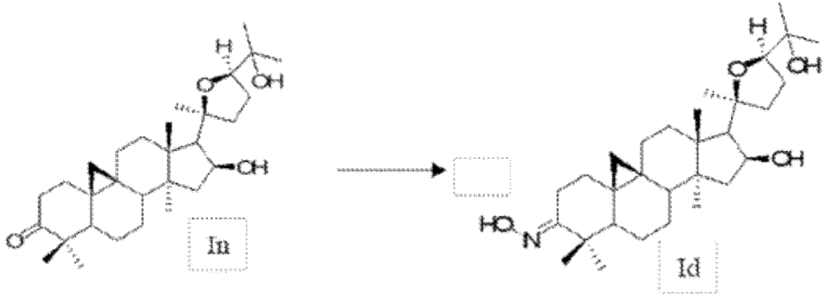

11. A guayulin compound according to claim 5, **characterized in that** the synthesis of the compound **(Ie)** is carried out in accordance with the following steps: a solution of 100 mg of In in 4 ml of acetic acid is treated at 0-5 °C with chromium trioxide (100 mg) in 0.3 ml of water. After 1 hour, the mixture is left at room temperature and subsequently extracted with AcOEt (3x). The organic phase is processed in a conventional way to obtain the product **Ie**

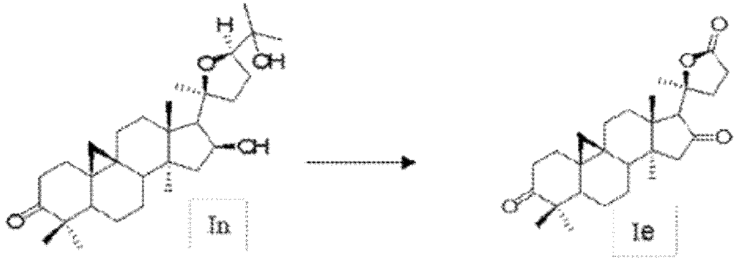

12. A guayulin compound according to claim 5, **characterized in that** the synthesis of the compound **(If)** is carried out in accordance with the following steps: a mixture of 200 mg of In, 60.2 mg of sodium acetate and 5 ml of acetic anhydride are heated at reflux temperature for 18 hours; subsequently, the mixture is poured into an Erlenmeyer flask containing 50 g of ice and stirred for 15 minutes; extracted with AcOEt (3x). The organic phase is dried and concentrated under reduced pressure to obtain a semi-solid. The product is recrystallized (hexane/AcOEt) to obtain acetate **If**

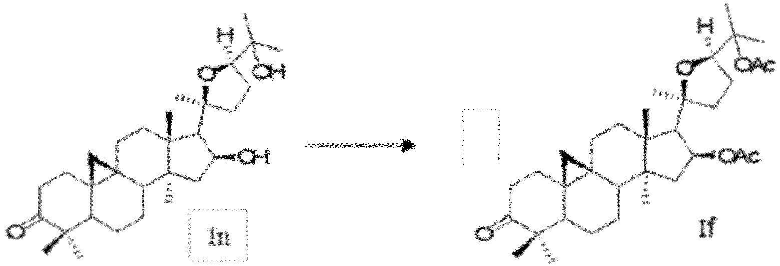

13. A guayulin compound according to claim 12 **characterized in that** the synthesis of the compound **(Ig)** is carried out in accordance with the following steps: 200 mg of diacetate In in 6.5 ml of pyridine are reacted with 95 mg of NH₂OH·HCl stirring at reflux temperature for 1.5 hours; subsequently, the reaction mixture is poured into a flask containing 50 g of ice and extracted with AcOEt (3x). The organic phase is washed 3 times with a 10% HCI solution and then with water. Recrystallization of the organic phase purifies the oxime **Ig**

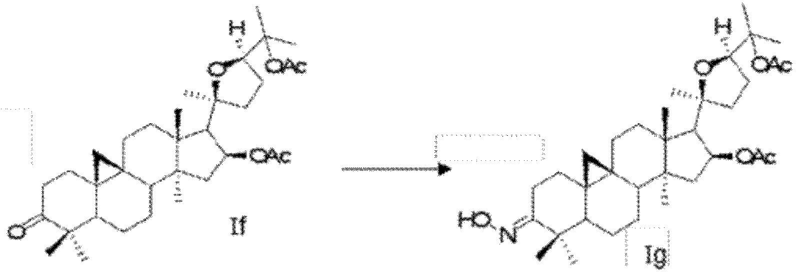

14. A guayulin compound according to claim 5, **characterized in that** the synthesis of the compound **(Ih)** is carried out in accordance with the following steps: a solution of 200 mg of In in 6 ml of dry pyridine, contained in Inert atmosphere, is added with 1 ml of ethyl formate (freshly distilled), 0.8 ml of a sodium solution in absolute MeOH (0.44 g/6 ml). The reaction is kept under stirring at room temperature for 8 to 12 hours until the appearance of an ocher color and/or the formation of an insoluble precipitate; subsequently the reaction mixture is placed in a cold solution of 3 ml of acetic acid in 27 ml of water; the precipitate is extracted with methylene chloride. The organic phase is washed with water and extracted with a 2% potassium hydroxide solution. The basic extract is washed with ether, acidified with glacial acetic acid, and finally extracted with methylene chloride. The final methylene chloride phase is dried and concentrated under reduced pressure to obtain an impure semi-solid product, purifying by preparative layer chromatography to obtain the formylated derivative **Ih.**

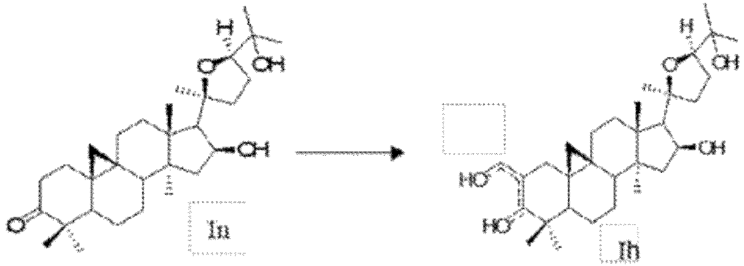

15. A guayulin compound according to claim 14, **characterized in that** the synthesis of compound **(Ii)** is carried out according to the following steps: a solution of 60 mg of the formylated derivative **Ih** in 5 ml of glacial acetic acid , with stirring, it is reacted for 2 hours with 30 mg of hydroxylamine hydrochloride at reflux temperature; subsequently the reaction mixture is poured into an Erlenmeyer flask containing 50 g of ice and extracted with AcOEt. The organic phase is washed with a 5% sodium bicarbonate solution (3x) and with water. The organic phase is dried and concentrated under reduced pressure to obtain an impure semi-solid, subsequently the semi-solid is purified by means of column chromatography to obtain isoxazole **Ii**

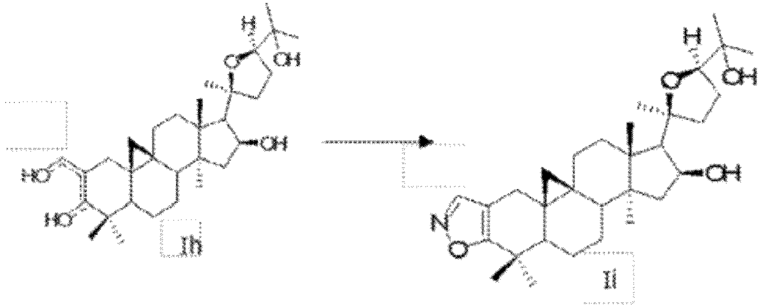

16. A guayulin compound according to claim 10, **characterized in that** the synthesis of the compound (Ij) is carried out in accordance with the following steps: to 100 mg of In oxime dissolved in CHCl₃, 0.5 mL of trifluoroacetic anhydride is added slowly at 0°C, the reaction mixture is stirred constantly at 25°C for 18 min; subsequently the reaction mixture is evaporated under reduced pressure. From this reaction, 16-trifluoroaxetoxy-lactam of In is obtained, a solution of potassium carbonate in methanol was added and stirred for 15 min. at room temperature, the solution is subsequently filtered, and the solvent is evaporated under reduced pressure. The reaction product is purified by column chromatography with polarity of 2:1 Hex:AcOEt, obtaining **Ij.**

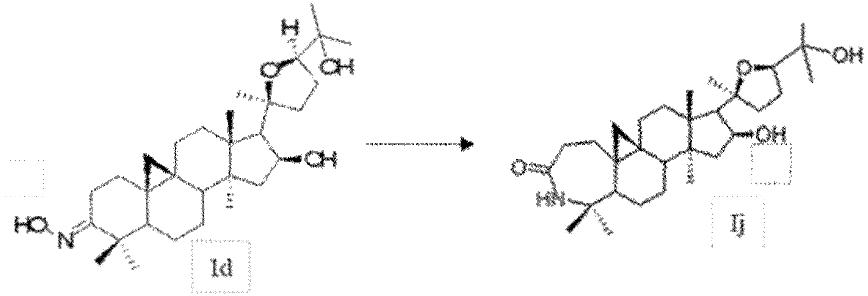

17. A guayulin compound/argentatines according to claim 5, **characterized in that** the synthesis of the compound **(Ik)** is carried out in accordance with the following steps: 200 mg of In are treated with 170 mg m-chloroperoxybenzoic acid for 3 hours, to obtain 180 mg of a white solid **Ik.**

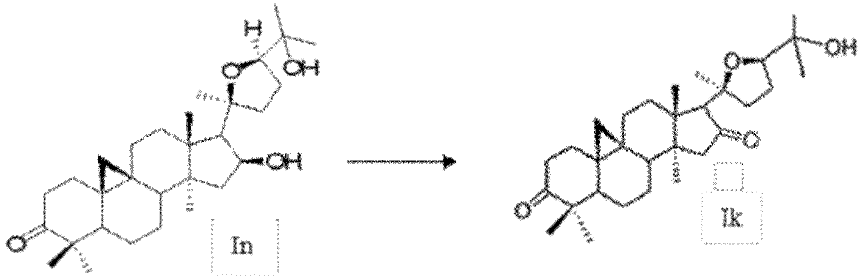

18. A guayulin compound according to claim 5, **characterized in that** the synthesis of the compound (II) is carried out in accordance with the following steps: 100 mg of In are treated with 80 mg sodium borohydride (Na8H₄) obtaining a white solid **II.**

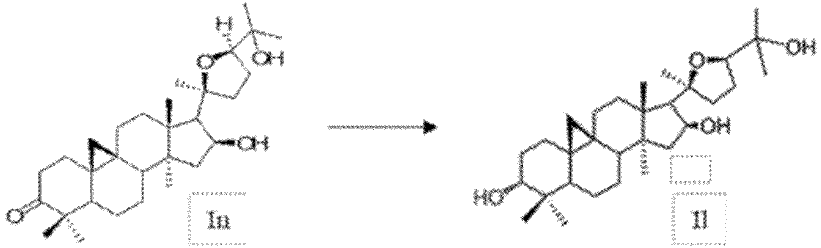

19. A guayulin compound according to claim 5, **characterized in that** the synthesis of the compound **(Im)** is carried out in accordance with the following steps: 0.15 mmol of hexadecanoyl chloride, previously obtained and in inert atmosphere, is added with 0.22 mmol of In, dissolved in 4 ml of dry dichloromethane. The reaction is stirred for 30 minutes; later, 15 mL of ethyl acetate was added, and it was placed in a separatory funnel. The organic phase is washed three times with distilled water and three times with a saturated solution of NaHCO₃, dried with anhydrous Na₂SO₄ and concentrated under reduced pressure. The reaction mixture is subject to silica open and packed column chromatography. The elution mixture used is hexane-ethyl acetate (7:3). From fractions 3-8 a white amorphous solid **(Im)** is obtained.

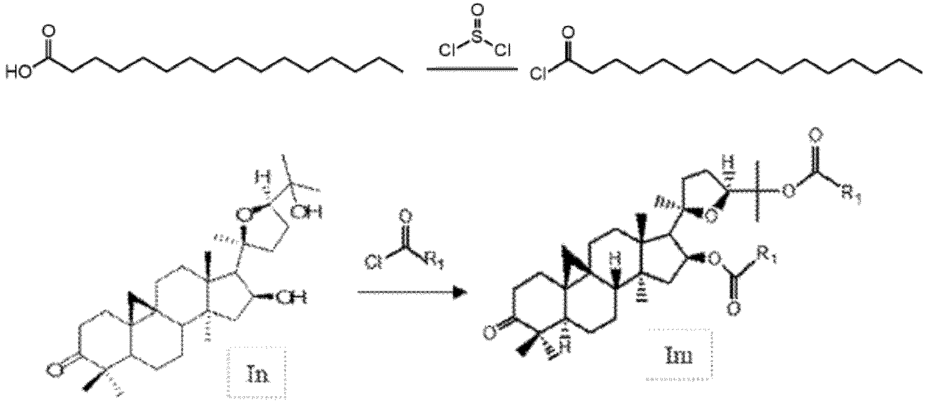

20. A guayulin compound according to claim 11, **characterized in that** the synthesis of the compound (I'a) is carried out in accordance with the following steps: a solution of 100 mg of le in EtOH is kept at reflux with 289 mg of potassium hydroxide for 40 min; subsequently the reaction is neutralized and 3,16-dioxo-25-nor-cycloartan-17-en-24-oic acid [I'a] is obtained.

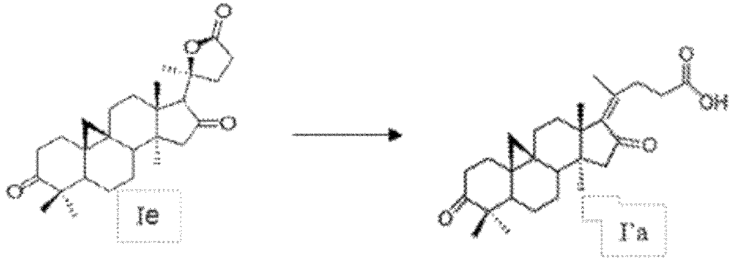

21. A guayulin compound according to any of the preceding claims, **characterized in that** they are active compounds that interfere with the inflammatory process and have antitumor activity in human cancer cell lines.

22. A guayulin compound according to any of the preceding claims, **characterized in that** the mechanism by which they exert an antitumor effect without generating cytotoxicity on healthy cells is through the induction of cellular senescence.
